# EUROPEAN PATENT SPECIFICATION

(11) **EP 3 209 328 B1**
(45) Date of publication and mention of the grant of the patent: **01.09.2021**
(21) Application number: 15791340.1
(22) Date of filing: 20.10.2015
(51) Int. Cl.: A61K 39/395, C07K 16/18, C07K 16/28

(54) **INHIBITORS OF LACTATE TRANSPORTERS FOR USE IN THE TREATMENT OF INFLAMMATORY DISEASES**
HEMMER VON LACTATTRANSPORTERN ZUR VERWENDUNG BEI DER BEHANDLUNG VON ENTZÜNDLICHEN ERKRANKUNGEN
INHIBITEURS DE TRANSPORTEURS DE LACTATE DESTINÉS À ÊTRE UTILISÉS DANS LE TRAITEMENT DE MALADIES INFLAMMATOIRES

(30) Priority: 20.10.2014 GB 201418626
(43) Date of publication of application: 30.08.2017
(73) Proprietor: Queen Mary University of London, London E1 4NS (GB)
(72) Inventor: MAURO, Claudio, London EC1M 6BQ (GB); HAAS, Robert, London EC1M 6BQ (GB); MARELLI-BERG, Federica, London EC1M 6BQ (GB)
(74) Representative: Bassil, Nicholas Charles
(86) International application number: PCT/GB2015/053125
(87) International publication number: WO 2016/063037

(56) References cited:
- WO-A1-2010/089580
- KR-A- 20120 123 748
- US-A1- 2004 072 746
- Anonymous: "NCT01791595 on 2013_10_07: ClinicalTrials.gov Archive", , 7 October 2013 (2013-10-07), XP055241680, Retrieved from the Internet: URL:https://clinicaltrials.gov/archive/NCT 01791595/2013_10_07 [retrieved on 2016-01-15]
- RENAUD LE FLOCH ET AL: "CD147 subunit of lactate/H+ symporters MCT1 and hypoxia-inducible MCT4 is critical for energetics and growth of glycolytic tumors", PROC NATL ACAD SCI, vol. 108, no. 40, 4 October 2011 (2011-10-04), pages 16663-16668, XP055241679,
- JOANNE R. DOHERTY ET AL: "Targeting lactate metabolism for cancer therapeutics", JOURNAL OF CLINICAL INVESTIGATION, vol. 123, no. 9, 3 September 2013 (2013-09-03), pages 3685-3692, XP055239122, US ISSN: 0021-9738, DOI: 10.1172/JCI69741
- CHICHE J ET AL: "The monocarboxylate transporter 1 (MCT1) and Hypoxia-induced MCT4 are key targets promoting tumor cell survival", EUROPEAN JOURNAL OF CANCER. SUPPLEMENT, PERGAMON, OXFORD, GB, vol. 6, no. 9, 1 July 2008 (2008-07-01), page 24, XP022833613, ISSN: 1359-6349, DOI: 10.1016/S1359-6349(08)71267-9 [retrieved on 2008-07-01]
- PIERRE SONVEAUX ET AL: "Targeting lactate-fueled respiration selectively kills hypoxic tumor cells in mice", JOURNAL OF CLINICAL INVESTIGATION, 20 November 2008 (2008-11-20), XP055242099, US ISSN: 0021-9738, DOI: 10.1172/JCI36843
- Robert Haas ET AL: "In the eye of the storm: T cell behavior in the inflammatory microenvironment", Am J Clin Exp Immunol, 15 June 2013 (2013-06-15), pages 146-155, XP055240437, Retrieved from the Internet: URL:http://www.ncbi.nlm.nih.gov/pmc/articl es/PMC3714175/pdf/ajcei0002-0146.pdf [retrieved on 2016-01-12]
- CLARE M MURRAY ET AL: "Monocarboxylate transporter MCT1 is a target for immunosuppression", NATURE CHEMICAL BIOLOGY, vol. 1, no. 7, 1 December 2005 (2005-12-01), pages 371-376, XP055241382, GB ISSN: 1552-4450, DOI: 10.1038/nchembio744
- ROBERT HAAS ET AL: "Lactate Regulates Metabolic and Pro-inflammatory Circuits in Control of T Cell Migration and Effector Functions", PLOS BIOLOGY, vol. 13, no. 7, 16 July 2015 (2015-07-16), page e1002202, XP055240411, DOI: 10.1371/journal.pbio.1002202
- GOPAL ET AL: "Cloning and functional characterization of human SMCT2 (SLC5A12) and expression pattern of the transporter in kidney", BIOCHIMICA ET BIOPHYSICA ACTA (BBA) - BIOMEMBRANES, ELSEVIER, AMSTERDAM, NL, vol. 1768, no. 11, 1 November 2007 (2007-11-01), pages 2690-2697, XP022338988, ISSN: 0005-2736, DOI: 10.1016/J.BBAMEM.2007.06.031
- I Tamai ET AL: "Participation of a proton-cotransporter, MCT1, in the intestinal transpor of monocarboxylic acids", Biochemical and Biophysical Research Communications, vol. 214, no. 2, 14 September 1995 (1995-09-14), pages 482-489, XP055484919,
- K. D. Rainsford: "Ibuprofen: pharmacology, efficacy and safety", INFLAMMOPHARMACOLOGY., vol. 17, no. 6, 21 November 2009 (2009-11-21), pages 275-342, XP055484915, NL ISSN: 0925-4692, DOI: 10.1007/s10787-009-0016-x
- Seema Rao ET AL: "Targeted Delivery of Anti-CTLA-4 Antibody Downregulates T Cell Function in Vitro and in Vivo", CLINICAL IMMUNOLOGY, vol. 101, no. 2, 1 November 2001 (2001-11-01), pages 136-145, XP055553868, US ISSN: 1521-6616, DOI: 10.1006/clim.2001.5119
- STANIMIROVIC DANICA ET AL: "Engineering and pharmacology of blood-brain barrier-permeable bispecific antibodies", ADVANCES IN PHARMACO, ELSEVIER, USA, vol. 71, 1 January 2014 (2014-01-01), pages 301-335, XP009188677, ISSN: 1557-8925, DOI: 10.1016/BS.APHA.2014.06.005
- C S D Roxburgh ET AL: "Cancer and systemic inflammation: treat the tumour and treat the host", British Journal of Cancer, vol. 110, no. 6, 18 February 2014 (2014-02-18), pages 1409-1412, XP055585294, GB ISSN: 0007-0920, DOI: 10.1038/bjc.2014.90

## Description

The present invention relates to the use of specific inhibitors of lactate transporters in the treatment of chronic inflammatory diseases. The invention also relates to methods of treatment of such diseases.

Chronic inflammation is a condition that is associated with a wide variety of diseases such as rheumatoid arthritis, osteoarthritis and cancer. Rheumatoid arthritis (RA) is one of the most common inflammatory diseases and a leading cause of chronic pain affecting approximately 0.5 to 1% of the population. This disease is characterized by chronic inflammation of the joints and is associated with synovitis and erosion of the cartilage and bone.

Although there are a number of treatment options available for RA, almost all of them have undesirable side effects. For example, non-steroidal anti-inflammatory drugs (NSAIDs) and disease-modifying anti-rheumatic drugs (DMARDs) both of which are currently the major forms of treatment for RA, have significant side effects ranging from gastrointestinal upset to liver and kidney damage.

There is thus a need for improved methods to treat chronic inflammatory diseases which are not associated with the disadvantages and problems mentioned above.

Recent studies have shed light on the interconnection between metabolism and immunity in multicellular organisms and their functional coordination for an effective establishment and resolution of immune responses. Imbalance of this delicate signalling network might lead to non-resolving inflammation and consequently to the development of chronic inflammatory disorders.

T-cells play a major role in the inflammatory process via both their cytolytic activities and the production of pro- and anti-inflammatory cytokines, which regulate immune responses. Upon antigen recognition by the T-cell receptor (TCR), downstream signalling events in naive T-cells lead to activation, proliferation and differentiation into effector T-cells. To maintain adequate supply of macromolecules (e.g. amino acids, nucleotides and fatty acids) during growth, T-cells undergo a metabolic switch from oxidative phosphorylation to aerobic glycolysis that is driven by signalling events generated by the TCR and the costimulatory molecule CD28. The metabolic machinery is also likely to directly affect T-cell migratory events, as T-lymphocytes continuously recirculate between different microenvironments (e.g. blood, lymphoid tissues and peripheral tissues), which might in turn modulate T-cell metabolism. In these "milieus", they are exposed to different nutrient availability and oxygen (O2) tension, and must adapt their metabolic pathways to effectively mediate immune responses. The direct effect of metabolism on the trafficking ability of T-cells, however, is yet to be investigated (Mauro C, Marelli-Berg FM (2012) Front Immunol 3: 173). Pro-inflammatory chemokines such as CXCL10 are produced in response to inflammatory stimuli and attract effector T-cells to the site of inflammation to fulfil their effector functions. Inflammatory sites however, are "harsh" micro-environments enriched with factors released by cellular components of the inflammatory infiltrates and the injured tissue itself that might affect the behaviour of effector T-cells and influence the outcome of the immune response.

Lactate has long been considered a "waste" by-product of cell metabolism, and it accumulates at sites of inflammation. Recent findings have identified lactate as an active metabolite in cell signalling although its effects on immune cells during inflammation are largely unexplored.

Use of a MCT2 inhibitor in the treatment of cancers expressing MCT1 over MCT4 has been described (WO 2010/089580 A1). ClinicalTrials.gov Identifier: NCT01791595 describes a phase I clinical trial of AZD3965 in patients with advanced cancer. The CD147 subunit of lactate/H⁺ symporters MCT1 and hypoxia-inducible MCT4 has been described as critical for energetics and growth of glycolytic tumours (Le Floch et al. (2011) PNAS 109: 40). Targeting lactate metabolism for cancer therapeutics has been described (Doherty JR and Cleveland JL (2013) J Clin Inv 123: 9). The monocarboxylate transporter 1 (MCT1) and Hypoxia-induced MCT4 have been described as key targets for promoting tumour cell survival (Chiche J et al. (2008) Eur J Can 6: 9). Targeting lactate-fuelled respiration has been reported to selectively kill hypoxic tumor cells in mice (Sonveaux et al. (2008) J Clin Inv 118: 12). A composition comprising phloretin for inducing glutathione S-transferase and inhibiting cytochrome P450 enzyme has been described (KR 2012 0123748 A). Inhibitors of monocarboxylate transport have been described (US 2004/072746 A1). T-cell behaviour in the inflammatory environment has been described (Haas R et al. (2013) Am J Clin Exp Immunol 2: 2). Monocarboxylate transporter MCT1 has been described as a target for immunosuppression (Murray et al. (2015) Nature Chem Bio 13: 7). Lactate has been reported to regulate metabolic and pro-inflammatory circuits in control of T-cell migration and effector functions (Haas et al. (2015) PLOS Bio 13: 7). Cloning and functional characterisation of human Slc5a12 and the expression pattern of the transporter in the kidney has been described (Gopal et al. (2007) Biomembranes 1768: 11). Participation of a proton-cotransporter, MCT1, in the intestinal transport of monocarboxylic acids has been described (Tamai I et al. (1995) Biochem Biophys Res Comms 214: 2). Ibuprofen pharmacology, efficacy and safety has been described (Rainsford KD (2009) Inflammopharm 17: 6). Targeted delivery of an anti-CTLA-4 antibody has been reported to downregulate T-cell function *in vivo* and *in vitro* (Rao et al. (2001) Clin Immunol 101: 2). Engineering and pharmacology of blood-brain barrier-permeable bispecific antibodies has been described (Stanimirovic D (2014) Adv Pharmaco 71). Therapeutic intervention using anti-inflammatory agents for cancer-associated symptoms has been described (Roxburgh CSD and McMillan DC (2014) Brit J Can 110: 6).

The present inventors have surprisingly found that extracellular sodium lactate and lactic acid inhibit the motility of CD4+ and CD8+ T-cells respectively and that this selective control of T-cell motility is mediated via subtype-specific transporters (Slc5a12 and Slc16a1) that are selectively expressed by CD4+ and CD8+ subsets, respectively. This is a previously unknown feature that differentiates these two subsets. The inventors have shown that inhibition of these lactate transporters promotes the release of T-cells from the inflamed tissue.

Aspects of the invention for which protection is sought are set out in the appended claims.

Thus described herein is a method of treating an inflammatory disease in a subject comprising administering to the subject a therapeutically effective amount of an inhibitor of Slc5a12 and/or Slc16a1. Slc5a12 is also referred to as solute carrier family 5 (sodium/glucose cotransporter), member 12. Slc16a1 is also referred to as solute carrier family 16 (monocarboxylate transporter), member 1.

Inflammatory disease as used herein refers to a disease such as rheumatoid arthritis, osteoarthritis, inflammatory bowel disorder, atherosclerosis and psoriasis.

A key feature of the inflammatory microenvironment is the accumulation of lactate, a by-product of the glycolytic pathway. Depending on the pH, lactate exists as the protonated acidic form (lactic-acid) in a low pH environment or as sodium salt (sodium-lactate) at basic pH. In physiological conditions (pH 7.2), most of the lactate is deprotonated and is present in the negatively charged, biologically active form as lactate anion.

Monocarboxylate transporters (MCTs) are proton-linked transmembrane proteins responsible for the transport for monocarboxylic molecules such as lactate, pyruvate, branched-chain oxo acids derived from leucine, valine and isoleucine, and the ketone bodies acetoacetate, beta-hydroxybutyrate and acetate across the plasma membrane. Fischer et al. described the expression by human CD8+ cytotoxic lymphocytes of the monocarboxylate transporter Slc16a1 (also known as Mct1), which facilitates lactic acid uptake. Slc5a12 is the only sodium lactate transporter described so far.

The term "inhibit" as used herein may refer to the detectable reduction and/or elimination of a biological activity exhibited by the lactate transporters in the absence of the inhibitor.

The inhibitor is selected from the group consisting of antibodies, aptamers, intramers, RNAi (double stranded RNA) and anti-Slc16a1 and/or Slc5a12 antisense molecules. Preferably, the inhibitor is an antibody. An antibody inhibitor may be described as a blocking antibody.

The term "antibody" includes intact antibodies, fragments of antibodies, e.g., Fab, F(ab') 2 fragments, and intact antibodies and fragments that have been mutated either in their constant and/or variable region (e.g., mutations to produce chimeric, partially humanized, or fully humanized antibodies, as well as to produce antibodies with a desired trait, e.g., enhanced IL-13 binding and/or reduced FcR binding). The antibody may be polyclonal or monoclonal.

As used herein, the term "specific inhibitor" refers to any molecule which predominantly inhibits a particular monocarboxylate transporter. A specific inhibitor of Slc16a1 predominantly inhibits Slc16a1. A specific inhibitor of Slc5a12 predominantly inhibits Slc5a12.

Accordingly, also described herein is a method of treating an inflammatory disease in a subject comprising administering to a subject a therapeutically effective amount of a specific inhibitor of Slc16a1 or Slc5a12.

In another embodiment the specific inhibitor of Slc16a1 may be administered in combination with the specific inhibitor of Slc5a12. In an embodiment, the specific inhibitor of Slc5a12 is an antibody and the specific inhibitor of Slc16a1 is another antibody.

As disclosed herein, the inhibitor may be a bispecific molecule. A bispecific molecule generally refers to a molecule having two or more different binding specificities. As used herein, the term "binding specificity" refers to the selective affinity of one molecule for another such as the binding of antibodies to antigens, receptors to ligands, and enzymes to substrates. All molecules that bind to a particular entity are deemed to have binding specificity for that entity. Thus all antibodies that bind a particular antigen have binding specificity for that antigen, and all ligands that bind to a specific cellular receptor have binding specificity for that receptor.

Methods for making bispecific polypeptides are known in the art. Early approaches to bispecific antibody engineering included chemical crosslinking of two different antibodies or antibody fragments and quadromas. Quadromas resemble monoclonal antibodies with two different antigen binding arms. They are generated by fusing two different hybridoma cells each producing a different monoclonal antibody. The antibody with the desired bispecificity is created by random pairing of the heavy and light chain.

TriomAbs are bispecific, trifunctional antibodies with each arm binding to a different antigen epitope and the Fc domain binding to FcR-expressing cells such as NK cells or dendritic cells. They are produced by a quadroma cell line prepared by the fusion of two specific hybridoma cell lines which allows the correct association of the heavy and light chain of each specificity without production of inactive heteromolecules.

The antigen-binding portion may be based on an scFv fragment. In a classical antibody molecule, the two domains of the Fv fragment, VL and VH, are coded for by separate genes. However they can be joined, using recombinant methods, by a synthetic linker that enables them to be made as a single protein chain known as single chain Fv (scFv) in which the VL and VH regions pair to form monovalent molecules.

ScFv fragments can be made bispecific using a number of approaches. ScFv molecules can be engineered in the VH-VL or VL-VH orientation with a linker varying in size to ensure that the resulting scFv forms stable monomers or multimers. When the linker size is sufficiently small for example 3 to 12 residues, the scFv cannot fold into a functional monomer. Instead, it associates with another scFv to form a bivalent dimer. When the linker size is further reduced, trimers and tetramers can form.

Diabodies are dimeric scFvs where the VH and VL domains of two antibodies A and B are fused to create the two chains VHA-VLB and VHB-VLA linked together by a peptide linker. The antigen binding sites of both antibodies A and B are recreated giving the molecules its bispecificity. Single-chain diabodies (sc-diabodies) have an additional linker connecting the VHA-VLB and VHB-VLA fragments. Tandem scFv consists of two sc-diabodies connected by a flexible peptide linker on a single protein chain. Another bispecific scFv format, the bispecific T-cell engager (BiTE) consists of two scFv fragments joined via a flexible linker where one fragment is directed against a surface antigen and the other against CD3 on T cells. Mini-antibodies are generated by the association of two scFv fragments through modified dimerization domains using a leucine zipper.

The scFv-Fc antibody is an IgG-like antibody with human IgG1 hinge and Fc regions (CH2 and CH3 domains). Each scFv arm can have a different specificity making the molecule bispecific. One method of generating an scFv-Fc heterodimer is by adopting the Knobs-into-Holes technology. Knobs are created by replacing small amino side chains at the interface between CH3 domains with larger ones, whereas holes are constructed by replacing large side chains with smaller ones. The bispecific molecule may be an scFv. The bispecific molecule may be a bispecific antibody, in particular a bispecific human antibody.

The bispecific molecule may have one binding specificity for a monocarboxylate transporter and the other binding specificity for a tissue specific antigen. Binding of the bispecific molecule to the monocarboxylate transporter leads to the inhibition of the monocarboxylate transporter. The monocarboxylate transporter may be Slc5a12 or Slc16a1. The tissue specific antigen may be an antigen specific to the synovium. An example of a polypeptide which specifically targets the synovial microvasculature of arthritis patients is described in WO 2012/042270.

The anti-Slc5a12 antibody may be specific for the region comprising amino acids 583-613 from the C-terminal region of human SLC5A12 (NP_848593.2 GI:157671931 as of 15 March 2015). The anti-Slc16a1 antibody may be specific for the region comprising amino acids 202-263 from human SLC16A1 (XP_011547028.1 GI:768055870 as of 12 March 2015)

Other inhibitors of Slc16a1 include, but are not limited to, phloretin, α-cyano-4-hydroxycinnamate (CHC) and AR-C155858 (6-[(3,5-Dimethyl-1H-pyrazol-4-yl)methyl]-5-[[(4S)-4-hydroxy-2-isoxazolidinyl] carbonyl]-3-methyl-1-(2-methylpropyl)thieno [2,3-d]pyrimidine-2,4(1H,3H)-dione).

As used herein, a subject refers to an animal, for example a mammal, including a human being. An animal can include mice, rats, fowls such as chicken, ruminants such as cows, goat, deer, sheep and other animals such as pigs, cats, dogs and primates such as humans, chimpanzees, gorillas and monkeys. Preferably the subject is human.

The diseases which may be treated according to the methods described in the present invention are diseases associated with inflammation. Examples include, but are not limited to, rheumatoid arthritis, osteoarthritis, inflammatory bowel disorder, atherosclerosis and psoriasis. In a preferred embodiment the inflammatory disease is rheumatoid arthritis.

The inventors have shown that in humans the synovial fluid of patients with rheumatoid arthritis (RA) presents with elevated levels of lactate compared with non- inflammatory types of arthritis e.g. osteoarthritis (OA). Thus, in a further embodiment, the methods provided herein may be used to treat diseases associated with elevated levels of lactate at the site of inflammation, including, but not limited to, rheumatoid arthritis, atherosclerosis and cancer. In one embodiment the disease is rheumatoid arthritis.

Also described herein is a pharmaceutical composition comprising an inhibitor of Slc5a12 and/or Slc16a1. The pharmaceutical composition may comprise a combination of inhibitors of Slc5a12 and Slc16a1.

The pharmaceutical composition may comprise a specific inhibitor of Slc5a12, optionally with a specific inhibitor of Slc16a1. The pharmaceutical composition may comprise a specific inhibitor of Slc5a12 in combination with a specific inhibitor of Slc16a1.

Pharmaceutical compositions described herein are suitable for the treatment of inflammatory diseases. Examples include, but are not limited to, rheumatoid arthritis, osteoarthritis, inflammatory bowel disorder, atherosclerosis and psoriasis.

A pharmaceutical composition described herein may be presented in a form that is ready for immediate use. Alternatively, the composition may be presented in a form that requires some preparation prior to administration.

Pharmaceutical compositions described herein may be adapted for administration by any appropriate route, for example by the oral (including buccal or sublingual), topical (including buccal, sublingual or transdermal), or parenteral (including subcutaneous, intramuscular, intravenous, intraperitoneal or intradermal) route.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents.

Excipients which may be used for injectable solutions include water, alcohols, polyols, glycerine and vegetable oils, for example. The compositions may be presented in unit-dose or multidose containers, for example sealed ampoules and vials, and may be stored in a freeze-dried (lyophilized) condition requiring only the addition of the sterile liquid carried, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

A therapeutically effective amount is the dose sufficient to reduce inflammation. Doses for delivery and administration can be based upon current existing protocols, empirically determined, using animal disease models or optionally in human clinical trials. Initial study doses can be based upon animal studies set forth herein, for a mouse, for example.

Doses can vary and depend upon whether the treatment is prophylactic or therapeutic, the type, onset, progression, severity, frequency, duration, or probability of the disease to which treatment is directed, the clinical endpoint desired, previous or simultaneous treatments, the general health, age, gender, race or immunological competency of the subject and other factors that will be appreciated by the skilled artisan. The dose amount, number, frequency or duration may be proportionally increased or reduced, as indicated by any adverse side effects, complications or other risk factors of the treatment or therapy and the status of the subject. The skilled person will appreciate the factors that may influence the dosage and timing required to provide an amount sufficient for providing a therapeutic or prophylactic benefit.

In a first aspect the invention provides an antibody to Slc5a12 for use in the treatment of an inflammatory disease, wherein the antibody to Slc5a12 is a specific inhibitor of Slc5a12. The inflammatory disease may be any one of rheumatoid arthritis, osteoarthritis, inflammatory bowel disorder, atherosclerosis and psoriasis. In a preferred embodiment the inflammatory disease is rheumatoid arthritis. In another embodiment the invention provides an antibody to Slc5a12 in combination with an antibody to Slc16a1 for use in the treatment of an inflammatory disease, wherein the antibody to Slc5a12 is a specific inhibitor of Slc5a12 and the antibody of Slc16a1 is a specific inhibitor of Slc16a1. Also described herein is the use of an inhibitor of Slc5a12 or Slc16a1 in the manufacture of a medicament for use in the treatment of an inflammatory disease. In a second aspect the invention provides a kit of parts comprising an antibody to Slc5a12 or an antibody to Slc5a12 in combination with an antibody to Slc16a1, wherein the antibody to Slc5a12 is a specific inhibitor of Slc5a12 and the antibody to Slc16a1 is a specific inhibitor of Slc16a1. In an embodiment of the invention the kit is for use in the treatment of diseases associated with inflammation. In a preferred embodiment the kit is for use in the treatment of rheumatoid arthritis. The kit may include a sealed container containing the inhibitors of the invention as a lyophilized powder and a second container containing a solvent. The peptide may be freeze dried. Further components may be included with the solid or liquid part. Thus the kit may comprise a first container containing the peptide and a second containing isotonic saline, or a first container containing the peptide and mannitol and a second container containing sterile water. Prior to administration the solvent is added to the container containing solid component in order to give the solution for injection.

Preferred features for the second aspect of the invention are as for the first aspect *mutatis mutandis.*

The invention will now be further described by way of reference to the following Examples and Figures which are included for the purposes of reference only and are not to be construed as being limitations on the invention:

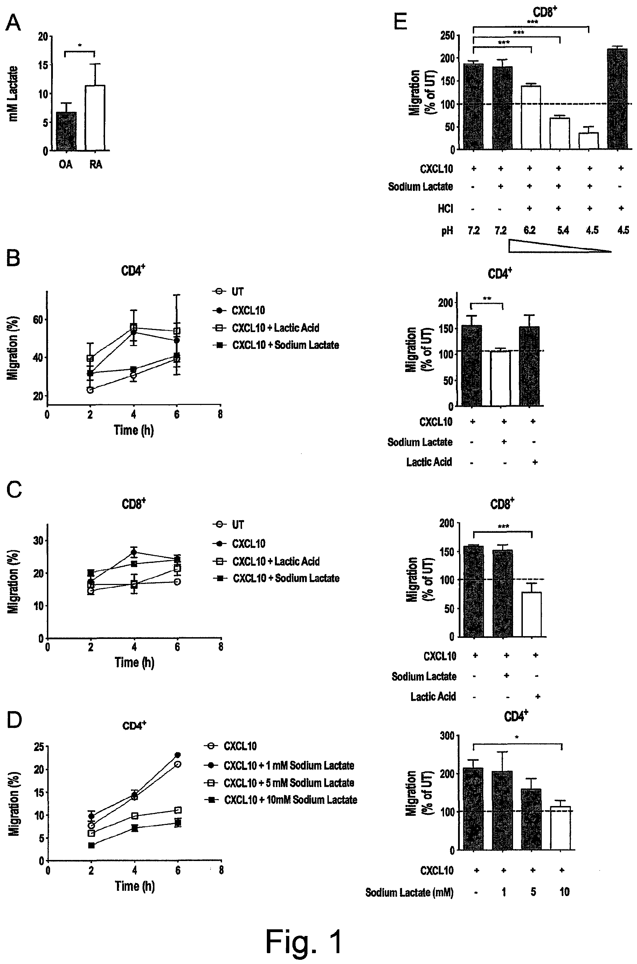

**Figure 1****: Lactate inhibits T-cell motility.** (A) Lactate measurements in the synovial fluid of osteoarthritis (OA) or rheumatoid arthritis (RA) patients. (B-C) *In vitro* chemotaxis of activated CD4⁺ (B) and CD8⁺ (C) T-cells towards CXCL10 (300ng/ml) in the presence of lactic-acid (10mM) or sodium-lactate (10mM) shown as kinetic (left panel) and 4h time point (right panel). (D) *In vitro* chemotaxis of activated CD4⁺ T-cells towards CXCL10 (300ng/ml) in the presence of increasing concentration of sodium-lactate shown as kinetic (left panel) and 4h time point (right panel). (E) *In* *vitro* chemotaxis (4h time point) of activated CD8⁺ T-cells towards CXCL10 (300ng/ml) in the presence of sodium-lactate (10mM) or HCI (pH 4.5) alone, or sodium-lactate in combination with increasing concentrations of HCI to obtain progressively reduced pH as indicated in figure. (A) OA, n=4 and RA n=8. (B right panel) n=4. (C-D right panel, E) n=3. (B-D left panel) Data is representative of three independent experiments. Values denote mean ± SD. *P<0.05; **P<0.01; ***P<0.001.

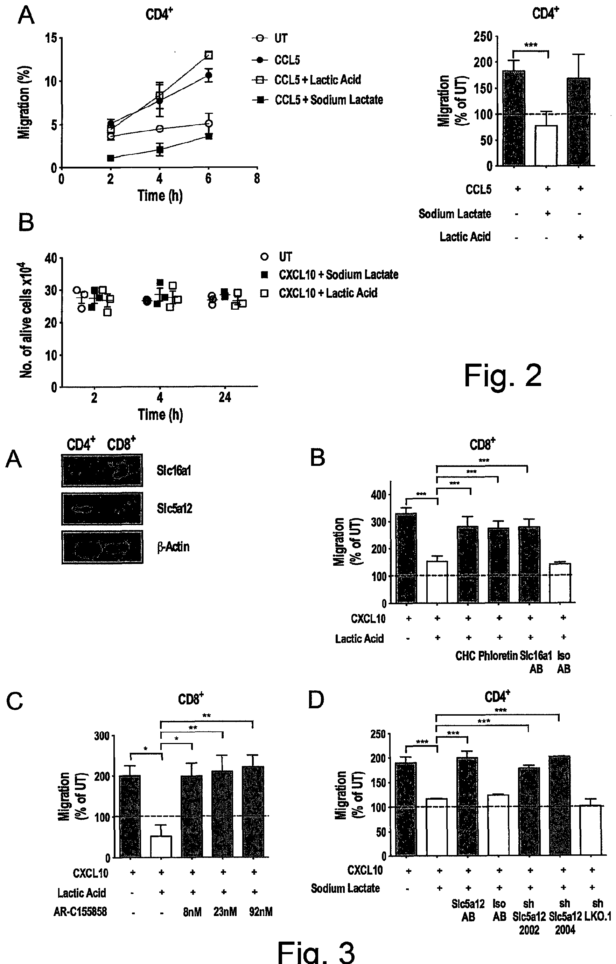

**Figure 2****: Lactate inhibits T-cell migration upon CCL5 stimulus and does not affect cellular viability.** (A) *In vitro* chemotaxis of activated CD4⁺ T-cells towards CCL5 (50ng/ml) in the presence of lactic-acid (10mM) or sodium-lactate (10mM), shown as kinetic (left panel) and 4h time point (right panel). (B) Total cell number of viable CD4⁺ T-cells treated with CXCL10 in the presence of lactic-acid (10mM) or sodium-lactate (10mM). (A left panel) Data is representative of three independent experiments. (A right panel, B) n=3. Values denote mean ± SD. ***P<0.001.
**Figure 3****: Sodium-lactate and lactic-acid act on CD4⁺ and CD8⁺ T-cell subsets, respectively, through specific cell membrane transporters.** (A) Total protein levels of the transporters Slc16a1 and Slc5a12 as assessed by Western Blot in activated CD4⁺ and CD8⁺ T-cell subsets. (B-D) *In vitro* chemotaxis (4 hour time point) of activated CD8⁺ T-cells towards CXCL10 (300ng/ml) in the presence of lactic-acid (10mM) alone, or in combination with CHC (425µM), phloretin (25µM) or anti-Slc16a1 antibody (2.5µg/ml) (B), or increasing concentrations of AR-C155858 as indicated in the figure (C), and activated CD4⁺ T-cells towards CXCL10 (300ng/ml) in the presence of sodium-lactate (10mM) alone, or in combination with an anti-Slc5a12 antibody (25µg/ml) or two specific sh-RNAs (D). An isotype control antibody has been included to control for antibody specificity (B, D) and a non-specific sh-RNA has been included to control for gene knockdown specificity (D). (B-D) n=3. Values denote mean ± SD. *P<0.05; **P<0.01; ***P<0.001.

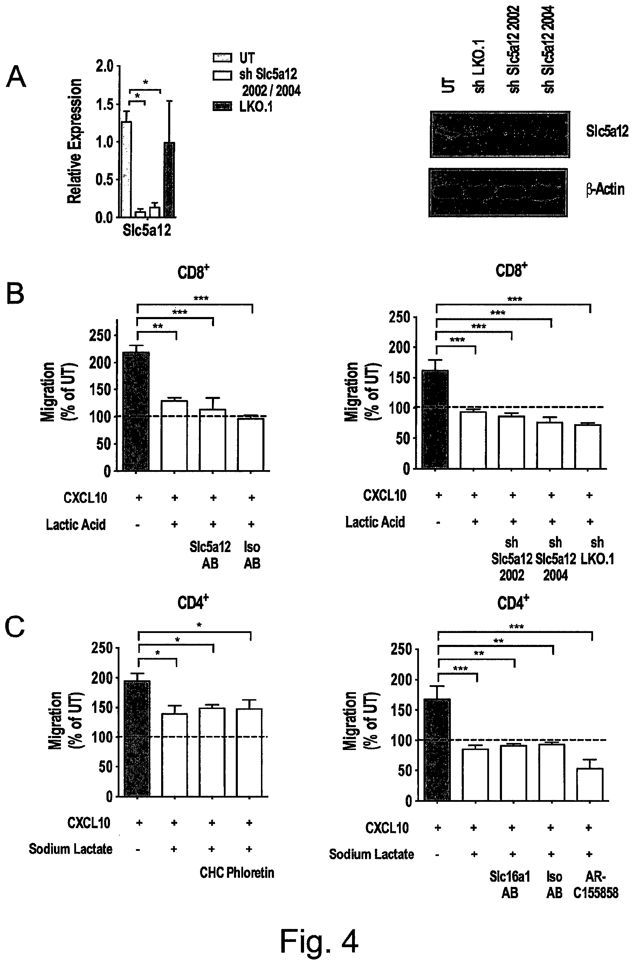

**Figure 4****: Inhibition of T-cell migration via blockade of lactate transporters is sub-type specific.** (A) Western blots and qRT-PCR with Slc5a12-specific primers and RNAs from activated CD4⁺ T-cells expressing the sh-RNAs shown. (B, C) *In vitro* chemotaxis (4h time point) of activated CD8⁺ T-cells towards CXCL10 in the presence of lactic-acid (10mM) alone or in combination with an anti-Slc5a12 antibody (25µg/ml) or an isotype control antibody (B left panel) and two specific sh-RNAs for Slc5a12 or a non-specific sh-RNA (B right panel), and activated CD4⁺ T-cells towards CXCL10 in the presence of sodium-lactate alone or in combination with CHC (425µM) or phloretin (25µM) (C left panel) and an anti-Slc16a1 (2.5□/ml) or an isotype control antibody, or ARC155858 (8nM) (C right panel). (A) Data is representative of three independent experiments. (B-C) n=3. Values denote mean ± SD.*P<0.05; **P<0.01; ***P<0.001.

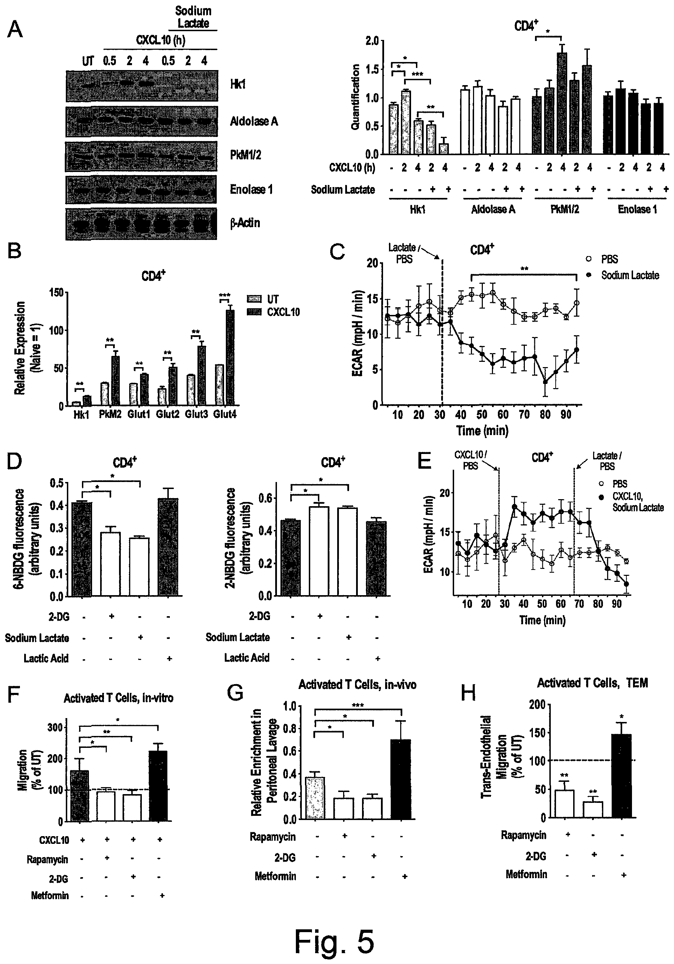

**Figure 5****: Basal and chemokine-induced aerobic glycolysis is required for CD4⁺ T-cell migration.** (A) Western blots with antibodies against Hk1, aldolase A, PkM1/2, enolase 1 and β-actin in activated CD4⁺ T-cells treated with CXCL10 (1000ng/ml) alone or in combination with sodium-lactate (10mM), or left untreated. Densitometric quantification of western blots denotes mean ± SD, n=3 (with biological replicates run in duplicate). *P<0.05; ***P<0.001. (B) Relative mRNA expression levels of Hk1, PkM2 and glucose transporters (Glut1, Glut2, Glut3, Glut4) in activated CD4⁺ T-cells 6h post-treatment with CXCL10 (1000ng/ml) as assessed by qRT-PCR. mRNA levels in naive CD4⁺ T-cells were set to 1. (C) ECAR trace of glycolytic activity expressed as mpH/min in activated CD4⁺ T-cells treated with sodium-lactate (10mM) or PBS. Vertical lines represent addition times of sodium-lactate or PBS, respectively. (D) Measurements of glucose uptake and flux in activated CD4⁺ T-cells pre-treated with 2-DG (1mM), sodium-lactate (10mM) or lactic-acid (10mM) and then incubated with the fluorescent probes 6-NBDG or 2-NBDG. (E) ECAR trace of glycolytic activity in activated CD4⁺ T-cells treated with CXCL10 (1000ng/ml) and sodium-lactate (10mM). Vertical lines represent the addition times of CXCL10, sodium-lactate and PBS. (F) *In vitro* chemotaxis (4h time point) towards CXCL10 (300ng/ml) of activated CD4⁺ T-cells pre-treated with Rapamycin (200nM), 2-DG (1mM) or Metformin (2mM). (G) Relative enrichment of i.v. injected activated CD4⁺ T-cells pre-treated with Rapamycin (200nM), 2-DG (1mM) or Metformin (2mM) and subsequently labelled with DDAO cell fluorescent dye in the peritoneal lavage of syngeneic recipient C57BL/6 mice i.p. injected with CXCL10 (120ng/mouse). (H) Spontaneous trans-endothelial migration (6h time point) of activated CD4⁺ T-cells in the presence of rapamycin (200nM), 2-DG (1mM) or metformin (2mM). (B-C, E) Data is representative of three independent experiments. (D, F, H) n=3. (G) n=4. Values denote mean ± SD. *P<0.05; **P<0.01; ***P<0.001.

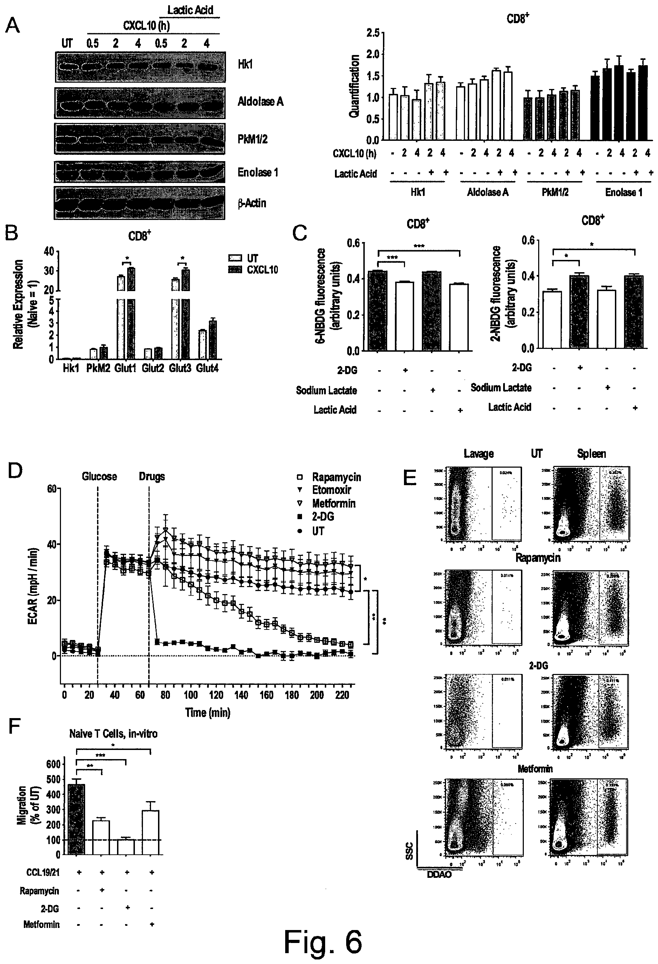

**Figure 6****: Glycolysis and chemotaxis in naive and activated CD4⁺ and CD8⁺ T-cells.** (A) Western blots with antibodies against Hk1, aldolase A, PkM1/2, enolase 1 and β-actin in activated CD8⁺ T-cells treated with CXCL10 (1000ng/ml) or left untreated. Densitometric quantification of western blots denotes mean ± SD, n=3 (with biological replicates run in duplicate). (B) Relative mRNA expression levels of Hk1, PkM2 and glucose transporters (Glut1, Glut2, Glut3 and Glut4) in activated CD8⁺ T-cells 6h post-treatment with CXCL10 (1000ng/ml) as assessed by qRT-PCR. mRNA levels in naive T-cells were set to 1. (C) Measurements of glucose uptake and flux in activated CD8⁺ T-cells pre-treated with 2-DG, sodium-lactate or lactic-acid and then incubated with the fluorescent probes 6-NBDG or 2-NBDG. (D) ECAR trace of glycolytic activity expressed as mpH/min in activated CD4⁺ T-cells treated with 2-DG (1mM), Rapamycin (200nM), Metformin (2mM) or Etomoxir (100µM). (E) Representative FACS dot plots of DDAO-labelled donor CD4⁺ T-cells collected from the peritoneal lavage and spleen of recipient mice, which correspond to the relative enrichment in peritoneal lavage shown in Fig. 5G. (F) *In vitro* chemotaxis (4h time point) towards the chemokines CCL19/21 (200ng/ml of each chemokine) of naive T-cells pretreated with Rapamycin (200nM), 2-DG (1mM) or Metformin (2mM). (B, D) Data is representative of three (B) and two (D) independent experiments. (C, F) n=3. Values denote mean ± SD. *P<0.05; **P<0.01; ***P<0.001.

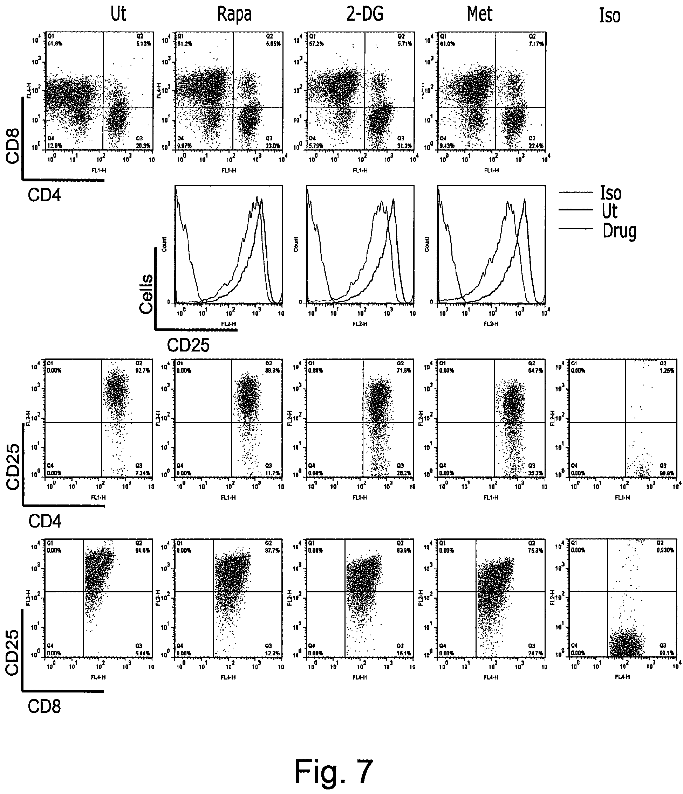

**Figure 7****: Metabolic drugs do not affect CD4⁺/CD8⁺ T-cell ratio and CD25 expression.** Representative FACS dot plots and histograms showing cell surface expression of CD4, CD8 and CD25 on activated T-cells treated with Rapamycin (200nM), 2-DG (1mM) or Metformin (2mM) as assessed by flow cytometry.

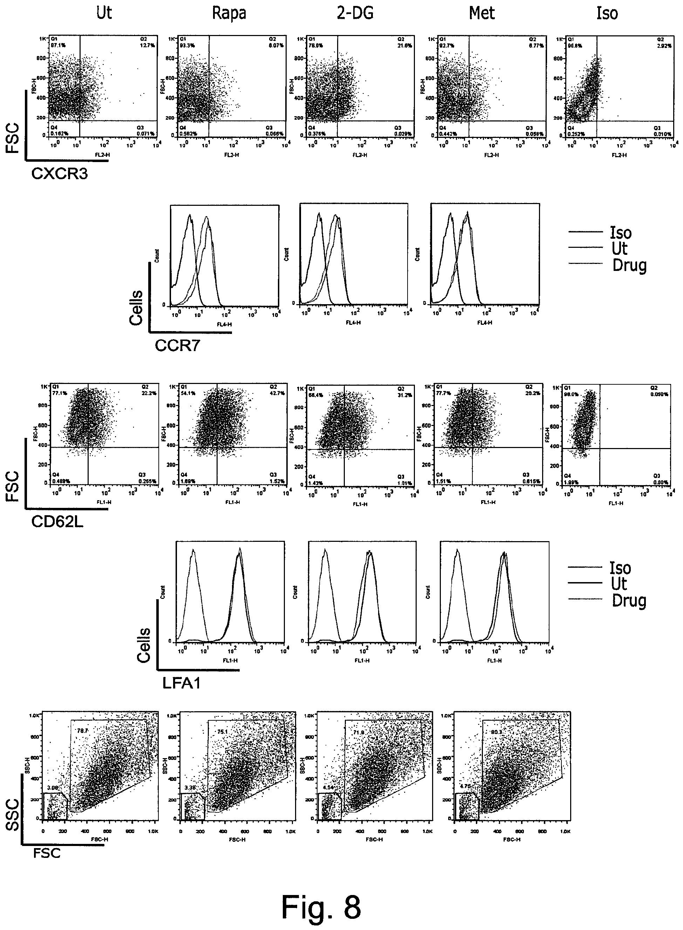

**Figure 8****: Metabolic drugs do not affect T-cell surface molecule phenotype and FSC/SSC profile.** Representative FACS dot plots and histograms showing FSC/SSC profile and cell surface expression of CXCR3, CCR7, CD62L and LFA-1 on activated T-cells treated with Rapamycin (200nM), 2-DG (1mM) or Metformin (2mM) as assessed by flow cytometry.

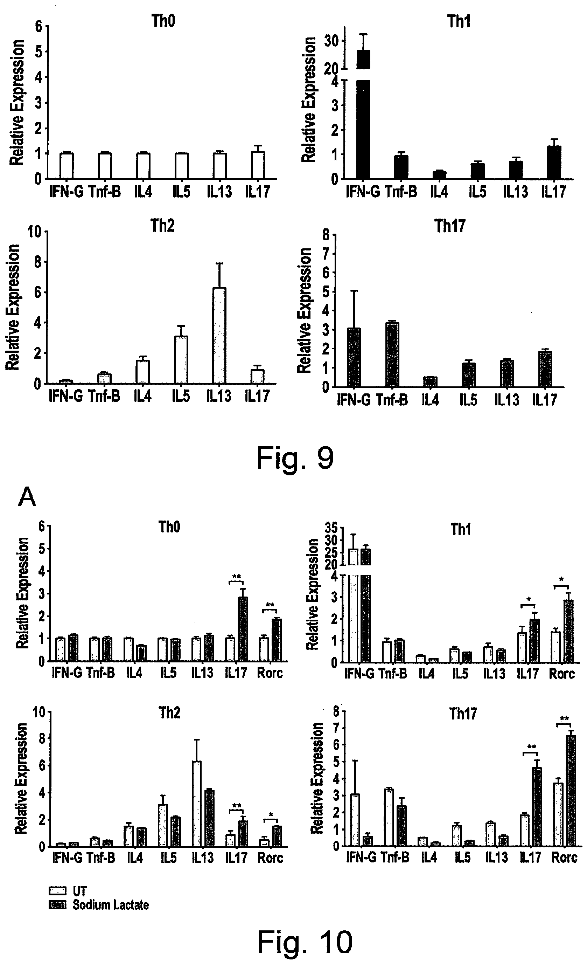

**Figure 9****: Cytokine expression profiles of Th0, Th1, Th2 and Th17 cell subsets.** Relative mRNA expression levels of the cytokines IFN-γ, Tnf-β, IL-4, IL-5, IL-13 and IL-17 as assessed by qRT-PCR. mRNA levels of each cytokine expressed by untreated Th0 cells were set to 1. Data is representative of three independent experiments.

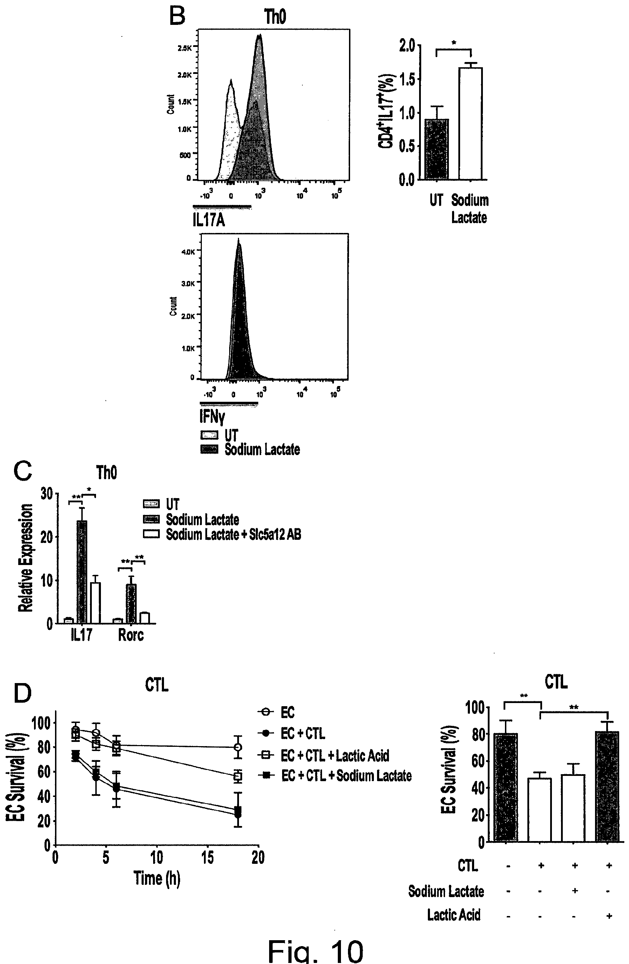

**Figure 10****: Lactate modulates effector T-cell functions.** (A) Relative mRNA expression levels of the cytokines IFN-γ, Tnf-β, IL-4, IL-5, IL-13 and IL-17 and of the transcription factor Rorc as assessed by qRT-PCR in CD4⁺ subsets Th0, Th1, Th2 and Th17 treated with sodium-lactate (10mM) or left untreated. mRNA levels of each cytokine expressed by untreated Th0 cells were set to 1. (B) Intracellular staining of IL-17A and IFN-γ in activated CD4⁺ T-cells treated with sodium-lactate (10mM) or left untreated. (C) Relative mRNA expression levels of IL-17 and Rorc in activated CD4⁺ T-cells treated with sodium-lactate alone or in combination with an anti-Slc5a12 antibody. mRNA levels of untreated T-cells were set to 1. (D) Cell survival of allogeneic endothelial cells in the presence of CD8⁺ cytotoxic T-cells and lactic-acid (10mM) or sodium-lactate (10mM) shown as kinetic (left panel) and 6h time point (right panel). (A, C, D left panel) Data is representative of three independent experiments. (B, D right panel) n=3. (A-D) Values denote mean ± SD. *P<0.05; **P<0.01.

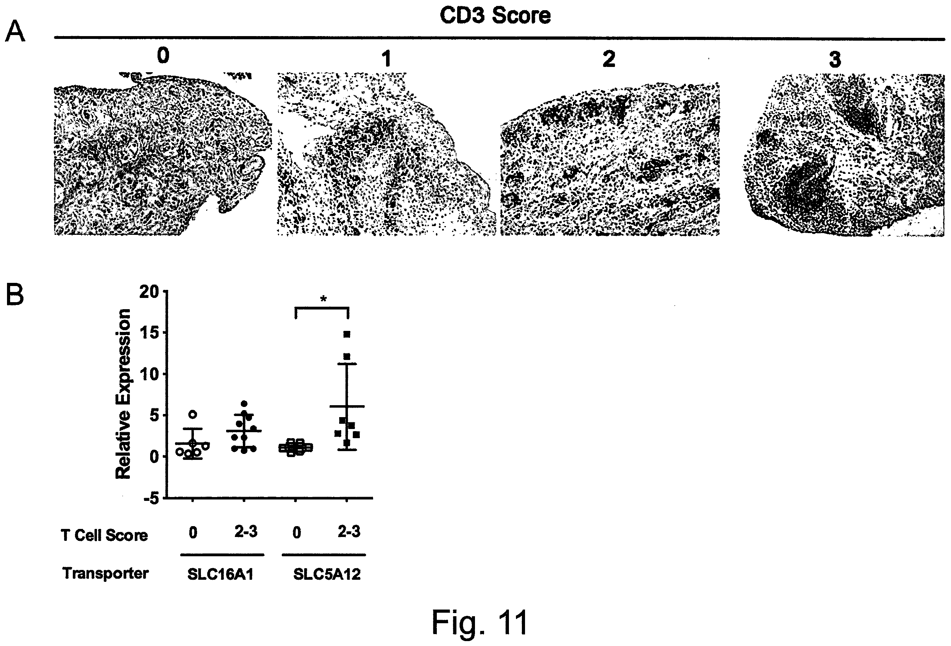

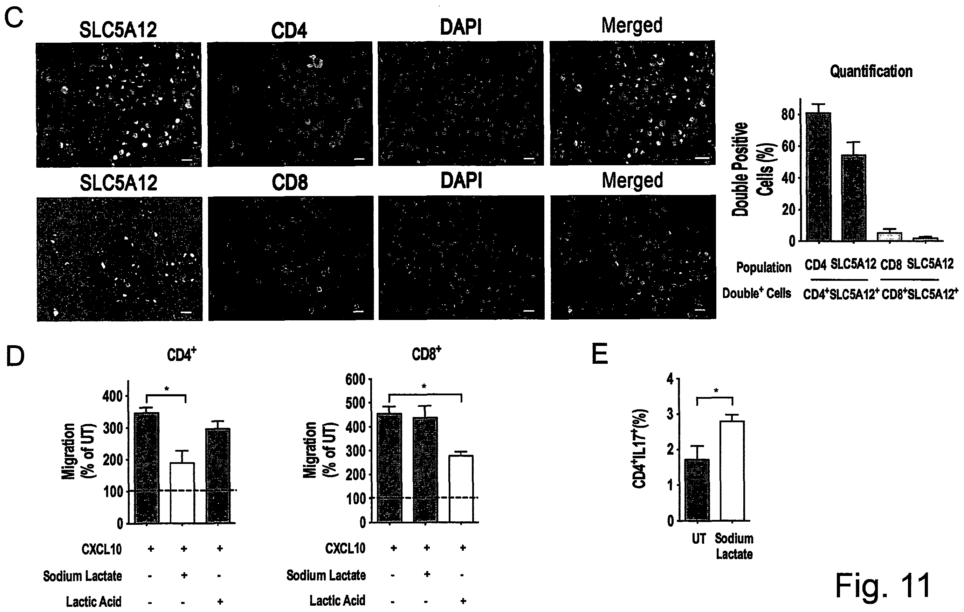

**Figure 11****: High Slc5a12 expression in RA in humans.** (A) Representative images of RA synovial tissues stained for CD3 displaying progressively higher degree of T-cell infiltration as quantified using a semi-quantitative score from T0 (absence of infiltrating T-cells) to T3 (large number of infiltrating T-cells organizing in ectopic follicles) as shown in Croia C et al. Ann Rheum Dis 2013. (B) Relative mRNA expression levels of Slc16a1 and Slc5a12 in the synovial fluid isolated from the joints of RA patients. Samples are grouped based on their T-cell score as described in A. Values denote mean ± SD, (T0) n=6 and (T2-3) n=7. *P<0.05. (C) Double immunofluorescence staining for Slc5a12 and CD4 or CD8 in the synovial tissue of RA patients. Slc5a12 (green) is highly expressed within the RA synovia in the presence of a high degree of CD4⁺ (red) T-cell infiltration. Merging (yellow) of the green and red channels demonstrates that Slc5a12 is selectively expressed by CD4⁺ but not CD8⁺ infiltrating T-cells. Quantification of the % double positive cells is provided upon counting positive cells (single and double positive for each marker) in at least 6 images per condition. Columns represent % of double positive CD4⁺ Slc5a12⁺ population within the CD4⁺ or Slc5a12⁺ cells and % of double positive CD8⁺ Slc5a12⁺ population within the CD8⁺ or Slc5a12⁺ cells. Scale bars: 50 µm. (D) *In vitro* chemotaxis (4h time point) of activated human CD4⁺ and CD8⁺ T-cells towards CXCL10 (300ng/ml) in the presence of lactic-acid (10mM) or sodium-lactate (10mM). (E) Intracellular staining of IL-17A in activated human CD4⁺ T-cells treated with sodium-lactate (10mM) or left untreated. (D) n=3. (E) n=4. Values denote mean ± SD. *P<0.05.

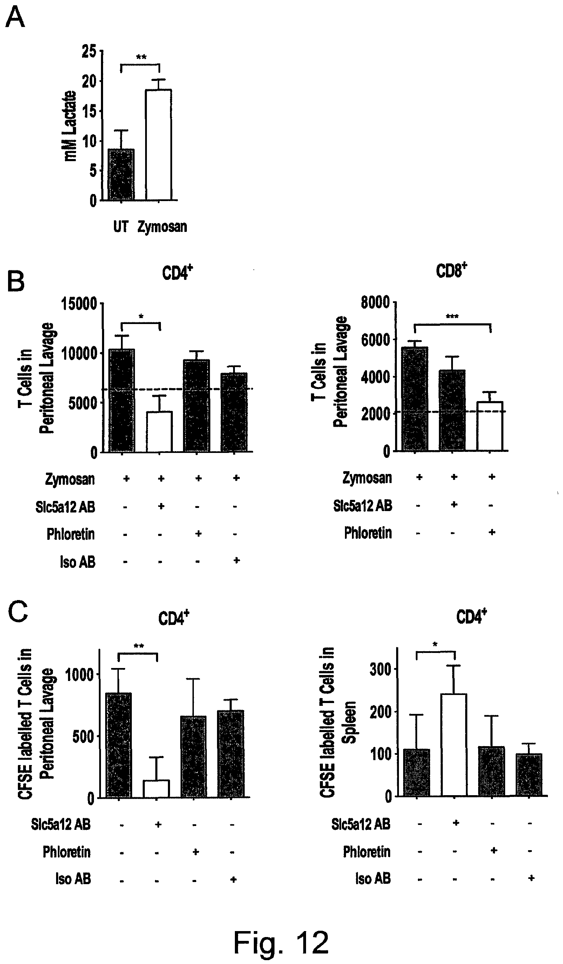

**Figure 12****: Inhibition of lactate transporters promotes the release of T-cells from the inflamed site in zymosan-induced peritonitis.** (A) Lactate levels in the peritoneum of zymosan-treated mice. (B) Number of CD4⁺ and CD8⁺ T-cells, respectively, in the peritoneal lavage of C57BL/6 mice injected i.p. with zymosan (1mg/mouse) to induce peritonitis, and 5 days later i.p. treated with phloretin (50µM), an anti-Slc5a12 antibody (5µg/ml) or an isotype control antibody. (C) Number of CFSE-labeled activated CD4⁺ T-cells in the peritoneal lavage (left panel) or spleen (right panel), respectively, of C57BL/6 mice injected i.p. with zymosan (1mg/mouse), then i.p. treated with phloretin (50µM), an anti-Slc5a12 specific antibody (5µg/ml) or an isotype control antibody. (A-C) n=3 or more. Values denote mean ± SD. *P<0.05; **P<0.01; ***P<0.001.

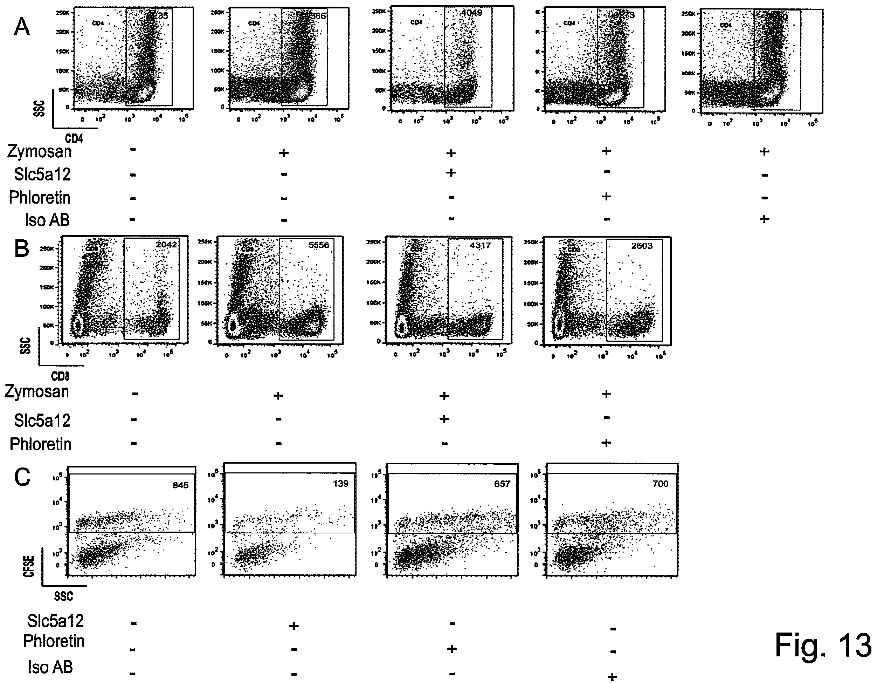

**Figure 13****: FACS dot plots of *in vivo* peritonitis model.** (A, B) Representative peritoneal lavage FACS dot plots of activated CD4⁺ (A) and CD8⁺ (B) T-cells of C57BL/6 mice injected i.p. with zymosan to induce peritonitis, and 5 days later treated with Slc5a12 specific antibody (5µg/ml), phloretin (50µM) or isotype control antibody, which correspond to the CD4⁺ and CD8⁺ T-cells in the peritoneal lavage shown in Fig. 12B. (C) Peritoneal lavage FACS dot plots of adoptively transferred CFSE-labeled activated CD4⁺ T-cells, which are representative of the analyses shown in Fig. 12C.

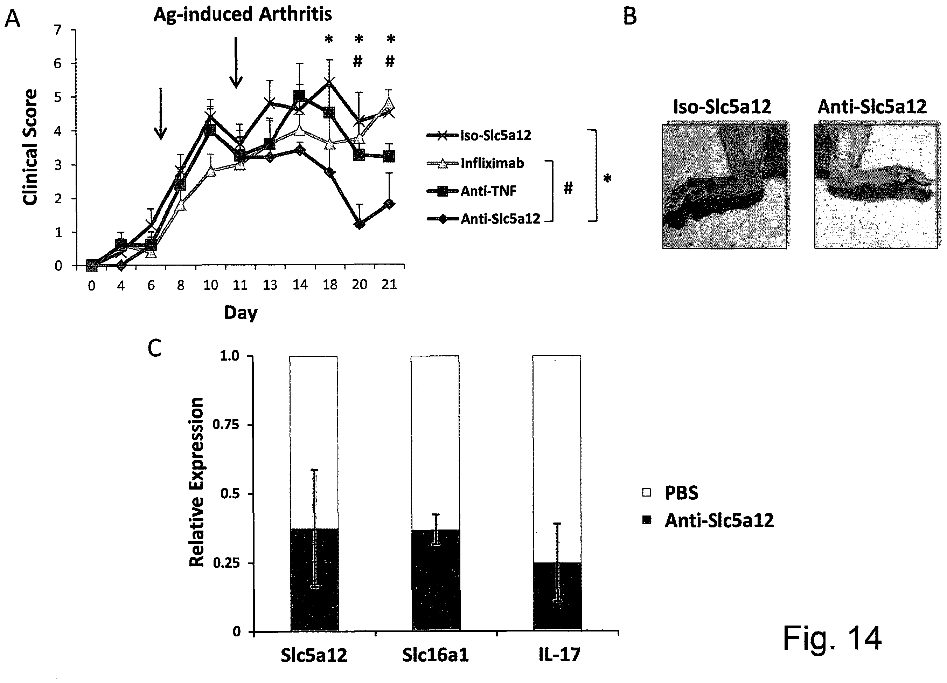

**Figure 14****: Anti-Slc5a12 suppresses arthritis in a mouse model.** DBA/1 mice purchased from Charles River Laboratories were immunized s.c. with 20µg human G6PI synthetic peptide (hG6PI325-339; ThermoFisher Scientific) in CFA (Sigma-Aldrich). The indicated amount of peptide was mixed with CFA in a 1:1 ratio (v/v) and emulsified by sonication. For induction of arthritis 100µl of the emulsion was given s.c. at the base of the tail. (A) On day 7 - a time point at the onset of disease - and 11 post induction (as indicated by the arrows in the graph) mice were left untreated or treated sub-plantar into the rear paws with 20 microl of 0.1 mg/ml antibody; Infliximab (Remicade, Janssen Biologics), anti-TNF (BD bioscience, TN3-19.12), anti-Slc5a12 (abcam), Iso-TNF (BD biosciences) and Iso-Slc5a12 (abcam). The dose of antibody was the same for each treatment group. The development of disease was monitored daily by visually assessing the clinical score. A score of 0 indicates no clinical signs of arthritis; a score of 1 for each of the fingers, pad and ankle indicates swelling and redness. Maximum score for each paw is 7. A trained observer who was blinded to the immunization status of the mice performed the scoring. (B) Representative images of the paws at day 21 post-immunization with G6PI showing the effects of treatment of anti-Slc5a12 as compared to an Slc5a12 isotype control antibody (i.e. swelling, redness). (C) Relative mRNA expression levels of Slc5a12, Slc16a1 and IL-17 in knees and ankles isolated from mice treated sub-plantar into the rear paws with PBS or anti-Sl5a12. (A-C) Values denote mean ± SD, n=6. *P<0.05.

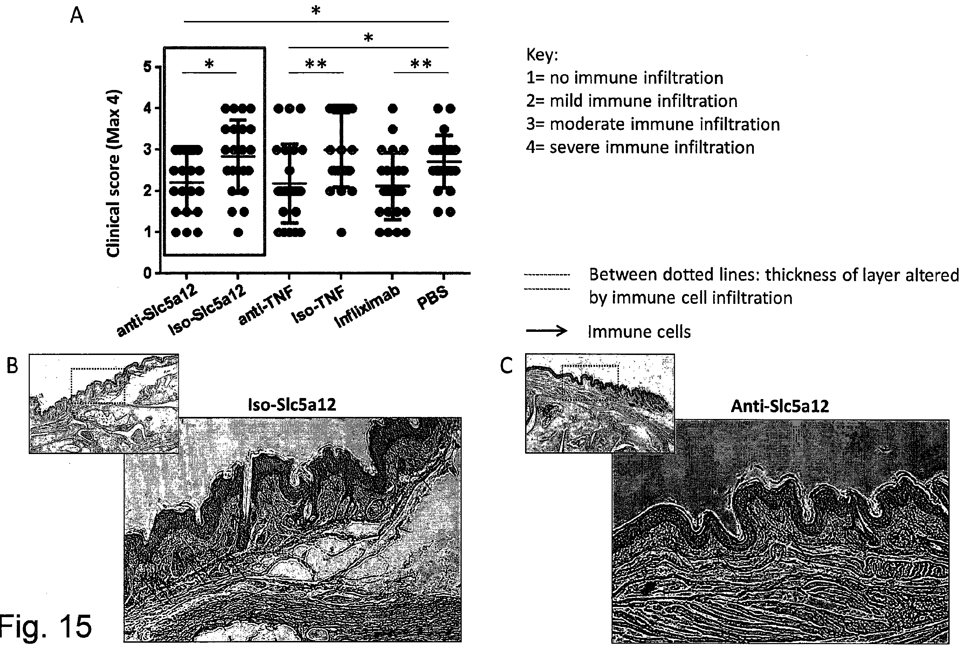

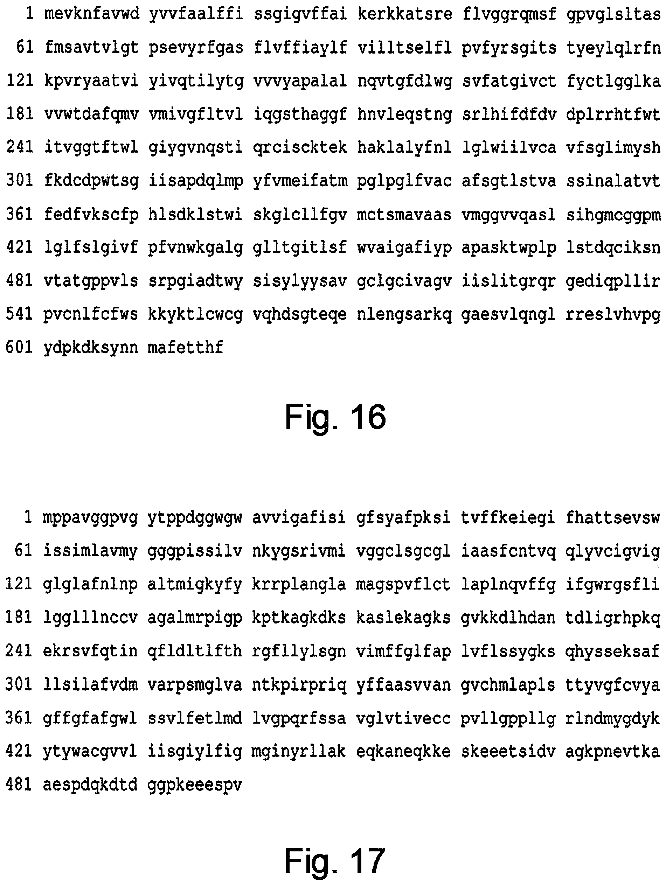

**Figure 15****: Anti-Slc5a12 reduces immune infiltrate in a mouse model of arthritis.** (A, B) Hematoxylin and eosin staining of ankles and pads sections taken from the experimental groups indicated in Fig. 14 showing drastically reduced immune infiltrate in the anti-Slc5a12 treatment group as compared to PBS and isotype control antibodies. Effect on immune infiltrate is comparable to anti-TNF treatment groups.

### Example 1: Lactate inhibits activated T-cell motility

To assess whether T-cell motility is affected by lactate, assays were performed whereby chemokinesis by T-cells activated for 5 days with anti-CD3 and anti-CD28 antibodies, and interleukin-2 (IL-2) was induced by the pro-inflammatory chemokine CXCL10 in the presence of 10mM lactic acid or sodium lactate, a concentration of lactate measured by the inventors in the synovial fluid of RA patients (Figure 1A) and found in a number of inflammatory sites. CD4+ T-cell chemokinesis was inhibited by sodium-lactate whereas that of CD8+ T-cells was inhibited by lactic-acid but not vice versa (Figures 1B, C).

As T-cell migration is activated by several chemokines, leading to different responses, the inventors also tested the effect of lactate on CCL5 (another inflammatory chemokine) -induced chemokinesis. CCL5-induced migration of CD4+ T-cells was decreased in sodium lactate- but not lactic acid-rich environment (Figure 2A), suggesting a broader action of lactate in chemokine induced signalling and downstream effects than was anticipated in experiments shown in Figures 1B and C. Migration of CD4+ T-cells upon sodium lactate treatment decreased with increasing concentration of sodium lactate with an EC50 of about 10mM sodium-lactate (Figure 1D). These concentrations of sodium-lactate and lactic-acid did not affect cellular viability (Figure 2B).

As acidification "per se" can affect cellular motility, to exclude a pH-dependent reduction of CD8+ T-cell migration, the inventors performed similar chemokinesis assays after buffering the culture media with bicarbonate or HEPES in the presence of lactic-acid to reach a neutral pH. In these conditions, chemokinesis of CD8+ T-cells was still impaired (Figure 1E). Conversely, adding to the culture media bicarbonate or HEPES without lactic-acid or acidifying the culture media alone with HCI had no effect on the migration of CD8+ T-cells (Figure 1E). This effect did not apply to CD4+ T-cells since their migration was not affected in lactic acid-rich medium (Figure 1B).

### Example 2: The effects of lactate action on different T-cell subsets are mediated by distinct transporters

The inventors next investigated the molecular basis of the differential and mutually exclusive responsiveness of CD4+ and CD8+ T-cells to sodium-lactate or lactic acid, respectively. Fischer et al. described the expression by human cytotoxic lymphocytes of the monocarboxylate transporter Slc16a1 (also known as Mct1), which facilitates lactic-acid uptake. Slc5a12 is the only sodium-lactate transporter described so far. The inventors found that murine CD8+ and CD4+ T-cells selectively express Slc16a1 and Slc5a12, respectively (Figure 3A), suggesting a specific functional role of each transporter on each T-cell subset. The inventors subsequently sought to confirm that the differently expressed lactate transporters were functional in T-cell chemokinesis inhibition.

Blockade of Slc16a1 on CD8+ T-cells with the selective inhibitors phloretin, α-cyano-4-hydroxycinnamate (CHC) and AR-C155858, or with a specific antibody restored chemokinesis of CD8+ T-cells exposed to lactic-acid (Figures 3B, C). Conversely, chemokinesis of CD4+ T-cells in sodium-lactate rich-media was recovered following selective inhibition of Slc5a12 on CD4+ T-cells with lentiviral-delivered, specific sh-RNAs or a specific antibody (Figures 3D and 4A). As expected, the anti-Slc5a12 antibody or sh-RNAs targeting Slc5a12 did not affect CD8+ T-cell migration (Figure 4B), nor did the Slc16a1 inhibitors phloretin, CHC and AR-C155858 or the anti-Slc16a1 antibody affect migration of CD4+ T-cells (Figure 4C).

### Example 3: Sodium-lactate limits basal and chemokine-induced aerobic glycolysis in CD4+ T-cells

The lactate transporters specificity (Figure 3A) and the T-cell insensitivity to lactate upon transporter inhibition (Figures 3B-D and 4B, C) suggest that the effects of lactate are mediated by intracellular signalling, possibly interfering with the cell metabolic machinery, and specifically with the glycolytic pathway, engaged downstream of chemokine receptor triggering. The inventors started by investigating the effect of CXCL10 treatment on the induction of glycolysis in CD4+ and CD8+ T-cells activated for 3 days with anti-CD3 and anti-CD28 antibodies, and interleukin-2 (IL-2). The inventors found that hexokinase 1 (Hk1) and pyruvate kinase (Pk) M2 were up-regulated in CD4+ T-cells early after CXCR3 engagement with CXCL10 at protein level and after 6 hours at mRNA level, suggesting the existence of multiple levels of regulation of the glycolytic pathway downstream of CXCR3 triggering, being both post-translational and transcriptional (Figures 5A, B). In addition, CD4+ T-cell exposure to CXCL10 led to increased protein expression of the enzymes enolase 1 and aldolase A (Figure 5A), and increased gene expression of glucose transporters (Figure 5B). Remarkably, sodium lactate inhibited the CXCL10-induced upregulation of glycolytic enzymes (Figure 5A). In contrast, CD8+ T-cells did not undergo major changes in the expression of glycolytic genes/proteins upon exposure to CXCL10 (Figures 6A, B). Since glycolysis is selectively activated in CD4+ T-cells upon CXCR3 triggering, the inventors then tested the effects of sodium-lactate on the glycolytic energy metabolism of CD4+ T-cells under basal conditions by measuring the extracellular acidification rate (ECAR) in the cell culture media of CD4+ T-cells in real time via the use of the Seahorse analyzer.

The inventors found that sodium lactate decreased the ECAR of CD4+ T-cells from an average of 14 mpH/min in the untreated control to a level of less than 5mpH/min (Figure 5C), indicating a decrease in glycolytic flux. In support of these data, the inventors treated CD4+ or CD8+ T-cells in the presence or absence of sodium-lactate or lactic-acid and measured glucose uptake and flux through glycolysis using the fluorescent probes 2-NBDG and 6-NBDG. 2-NBDG enters the glycolytic pathway, being phosphorylated by hexokinase and rapidly degraded to non-fluorescent products. In contrast, 6-NBDG cannot be phosphorylated by hexokinase and accumulates in the cytoplasm in its fluorescent form. The inventors show that sodium-lactate but not lactic-acid selectively blocks glucose uptake and flux through glycolysis in CD4+ but not CD8+ T-cells and vice versa (Figures 5D and 6C). The inventors next investigated whether sodium lactate was able to diminish glycolysis also upon CXCR3 engagement by CXCL10. Exposing CD4+ T-cells to CXCL10 raised the ECAR value for several time points, indicating that the glycolytic flux is increased in these conditions (Figure 5E). Adding sodium-lactate to the CXCL10-stimulated cells shut down glycolysis, as reflected by a drastic fall of ECAR (Figure 5E).

### Example 4: Basal and chemokine-induced aerobic glycolysis is required for CD4+ T cell migration both in vitro and in vivo

The down-regulation of glycolysis and the inhibitory effect on migration upon sodium-lactate exposure - lactate being a direct and indirect inhibitor of glycolysis - suggest that glycolysis is required for CD4+ T-cell migration. To test this hypothesis, the inventors treated activated CD4+ T-cells with inhibitors or activators of glycolysis and assessed their chemokinetic responses to CXCL10. Direct or indirect inhibition of glycolysis with the glucose analogue, 2-deoxyglucose (2-DG), or mTOR inhibitor, rapamycin (Figure 6D), caused a decrease in chemokinesis in vitro and in a well-established in vivo model of T-cell recruitment to the peritoneum (Jarmin et al 2008 J Clin Invest 118: 1154-1164) (Figures 5F, G and 6E). Conversely, activation of glycolysis using the electron transfer chain Complex I inhibitor, metformin (Figure 6D), increased chemokinesis towards CXCL10 both in vitro and in vivo (Figures 5F, G and 6E). Accounting for the specificity of the glycolytic measurements, etomoxir, an inhibitor of fatty acid oxidation, had only minor effects on glycolysis (Figure 6D).

Metabolic drugs interfering with glycolysis had similar effects on T-cell motility in spontaneous chemokinesis assays (i.e. independent of any pro-inflammatory chemokine stimulus), implying the role of this pathway in steady-state control of T-cell migration (Figure 5H). Importantly, exposure to the various metabolic drugs at the concentrations used did not affect the T-cell surface molecule phenotype (Figures 7 and 8). The importance of aerobic glycolysis in activation and function of T-cells has been shown previously, yet its potential impact on T-cell migration is still unexplored; thus we assessed the impact of glycolysis on migration of naive T-cells, which mainly rely upon oxidative metabolism for their homeostasis.

Similar to what was observed in activated T-cells, inhibition of glycolysis via 2-DG and rapamycin resulted in a decrease in naive T-lymphocyte motility (Figure 6F), suggesting a general control of T-cell migration via the glycolytic pathway. In contrast to activated T-cells, however, exposure to metformin reduced naive T-cell migration (Figure 6F), indicating the existence of different metabolic checkpoints between naive and activated T-cells. Overall, metabolic drugs did not affect T-cell survival of T-lymphocytes at the concentrations and in the experimental conditions used (data not shown).

### Example 5: Lactate modulates effector T-cell functions

To investigate whether sodium-lactate could affect CD4+ T-cell effector functions, the inventors induced polarization of CD4+ T-cells towards Th1, Th2 and Th17 subsets in the appropriate cytokine "milieus". The expected patterns of cytokine expression by differentiated Th subsets were confirmed at the mRNA level (Figure 9). The inventors then tested the effect of the presence of sodium-lactate on the release of cytokines by the different Th subsets in the same polarizing conditions. Gene expression analysis showed that treatment with sodium-lactate caused a significant up-regulation of IL-17 in all the Th subsets (Figure 10A).

Supporting these data, gene expression of Rorc, the signature transcription factor of Th17 cells, was also significantly elevated in all the Th subsets upon T-cell exposure to sodium-lactate (Figure 10A). Gene expression of Th1 and Th2 signature cytokines was either unmodified upon treatment with sodium-lactate or even reduced (i.e., IL-4, IL-5 and IL-13 in the Th17 subset; Figure 10A). Intracellular staining experiments confirmed the increased expression of IL-17 protein in CD4+ T-cells exposed to sodium-lactate as compared to cells left untreated (Figure 10B). Remarkably, preincubation with the antibody anti-Slc5a12 blocked the upregulation of IL-17 and Rorc genes induced by sodium-lactate (Figure 10C).

Cytotoxic T-cells (CTLs) differentiating from the CD8+ subset express and release cytolytic granules consisting of perforin/granzyme complexes, which promote the killing of target cells. To test whether these functions were affected by lactate, the inventors performed cytotoxicity assays with allogeneic endothelial cells. Similarly to the results obtained in migration assays (Figure 1C), lactic acid but not sodium-lactate inhibited the cytolytic activity of CTLs (Figure 10D).

### Example 6: Inhibition of lactate transporters promotes the release of T-cells from the inflamed tissue

The inventors have shown that in humans the synovial fluid of RA (inflammatory form of arthritis) presents with elevated levels of lactate compared with non-inflammatory types of arthritis (e.g. osteoarthritis [OA], Figure 1A). The rheumatoid synovial environment is paradigmatic of all the lactate-induced changes in T-cells, including entrapment, IL-17 secretion and loss of antigen responsiveness. The inventors therefore investigated the expression and cellular localization of Slc5a12 and Slc16a1 within the synovial tissue of 16 patients suffering from RA (Table 1, demographical data).

**Table 1**

| **Parameter** | | **Study Population (n = 16)** |
|---|---|---|
| Age (range) | | 46-76 |
| Gender | Female | 80 |
| | Male | 20 |
| Site | Large joint | 68 |
| | Small joint | 32 |
| Erosive (%) | | 63 |
| Treatment (%) | DMARDs | 81 |
| | Steroids | 15 |
| | Biologics | 61 |
| RF⁺ and/or CCP⁺ (%) | | 67 |

RA patients were stratified for the amount of CD3+ infiltrating T-cells using a semi-quantitative score (Figure 11A) as previously described in Croia et al (2013) Ann Rheum Dis 72: 1559-1568. Next, gene expression analysis was performed and it was found that Slc5a12 mRNA expression significantly increased in correlation with the T-cell score of the samples tested (Figure 11B, right). Albeit not significant, a trend towards increased Slc16a1 expression could also be observed in CD8+ T-cells (Figure 11B, left). In order to confirm *in vitro* data that Slc5a12 is expressed on CD4+ but not CD8+ T-cells (Figure 3A), the inventors performed double immunofluorescence for Slc5a12 and either CD4 or CD8. As shown in Figure 10C, within the RA synovial tissue Slc5a12 is abundantly and selectively expressed by CD4+ but not CD8+ T-cells. Enhanced expression of the Slc5a12 transporter by CD4+ T-cells in the RA synovia opens the possibility that this transporter might be mediating the migratory and functional changes that the inventors have previously described and that correlate with key features of T-cell infiltrates in RA.

Prompted by these results, the inventors sought to assess whether lactate promotes the retention of T-cells into inflammatory sites in vivo and whether inhibitors of the lactate transporters favour the release of T-cells from the inflamed site. The inventors used a well-established mouse model of zymosan-induced peritonitis, in which T-cells are recruited to the inflamed site 5 days after zymosan injection (Montero-Melendez et al 2011 Am J Pathol 179: 259-269). C57BL/6 mice were injected in the peritoneal cavity with zymosan (1mg/mouse) on day 0 or left untreated. On day 5, Phloretin, an anti-Slc5a12 antibody or an isotype control antibody were injected into the peritoneal cavity. 24 hours later, mice were sacrificed and the peritoneal lavage was harvested. Lactate levels and CD4+ and CD8+ T-cells in the peritoneum were increased significantly in the peritoneum of recipient animals (Figures 12A, B). Intraperitoneal injection of anti-Slc5a12 antibody caused a significant reduction of CD4+ T-cells in the peritoneum in comparison to an isotype control antibody, while having no effect on CD8+ T-cells (Figures 12B and 13A, B). In contrast, phloretin promoted a significant decrease of CD8+ T-cells in the peritoneum but did not show any effect on CD4+ T-cells (Figures 12B and 13A, B).

To establish that the decrease in T-cell localization to the peritoneal cavity was at least in part due to their increased release from this site, the inventors performed adoptive transfer experiments whereby CFSE-labelled CD4+ T-cells were co-injected with anti-Slc5a12 antibody, phloretin or an isotype control antibody in the peritoneal cavity of mice which had received zymosan (1 mg/mouse) 5 days before, to create an environment rich of lactate (Figure 12A). 24 hours after the intraperitoneal injection of CFSE-labelled CD4+ T-cells, mice were sacrificed, and peritoneal lavage and spleen were harvested. Injection with anti-Slc5a12 antibody but not phloretin or the isotype control antibody in the peritoneal cavity caused a selective reduction of adoptively transferred T-cells in the peritoneum and their accumulation in the spleen (Figures 12C and 13C).

### Example 7: Anti-Slc5a12 suppresses arthritis in a mouse model.

Finally, the inventors took advantage of a well-established mouse model of glucose-6-phosphate isomerase (G6PI)-induced arthritis (Schubert et al 2004 J Immunol 172: 4503-4509) to prove the principle that blocking the sodium lactate transporter Slc5a12 represents an innovative mechanism of action for the therapy of RA. Specifically, they showed that inhibiting Slc5a12 with specific polyclonal antibodies that are commercially available ameliorates the clinical scores and paw swelling (Figure 14A, B) in the murine model of G6PI-induced. Remarkably, the effects of anti-Slc5a12 antibodies compare well against standard anti-TNF therapies (Figure 14A).

### MATERIALS AND METHODS

All chemicals and reagents were purchased from Sigma-Aldrich & Co (UK), unless otherwise specified.

T-cell isolation, in-vitro activation and subset enrichment: T-cells were isolated from C57BL/6 murine lymph nodes and activated for 3 to 5 days with plate bound anti-CD3 and anti-CD28 antibodies (BioLegend), and IL-2 (PeproTech). CD4+ and CD8+ subsets were enriched with commercially available CD4+ and CD8+ T-cell isolation kits according to the manufacturer's instructions (Easysep, Invitrogen) either prior or post activation according to experimental settings. Chemokinesis assays: Chemokinesis assays were performed in 5µm transwell inlays. In some experiments, T-cells were pre-treated overnight with a number of drugs purchased from Calbiochem: Rapamycin (200nM), 2-DG (1mM), Metformin (2mM). In most experiments, 1 hour before the assay cells were incubated with lactic-acid (10mM) or sodium-lactate (10mM), either alone or in combination with Phloretin (25 µM), CHC (425µM), increasing concentrations of ARC155858, Slc5a12 specific antibody (25µg/ml) or Slc16a1 specific antibody (2.5µg/ml). 3x10⁵ lymphocytes were seeded in the upper transwell chamber; chemokines were added to the lower chamber: CXCL10 (300ng/ml), CCL5 (50ng/ml), CCL19/21 (200ng/ml of each chemokine). Migrated T-cells were counted with a hemocytometer 2, 4 and 6 hours after seeding and % of migrated cells was calculated.

RNA isolation and reverse transcription: RNA was isolated from 10⁶ cells or 10mg RA synovial tissue using commercially available kits (Qiagen) or Trizol (Life) according to the manufacturer's instructions and assessed for quality and quantity using absorption measurements. Reverse transcription to cDNA was performed according to the manufacturer's instruction (Applied Biosystems).

qRT-PCR: Gene expression analysis was done using SYBR Green Supermix (Biorad) in CFX connect light cycler (Biorad), according to the manufacturer's instructions. Gene relative expression was calculated using the ΔΔct method and normalized to a reference control (Rplp0). Primers for qRT-PCR were designed with the assistance of online tools (Primer 3Plus) using at least one exon junction binding site per primer pair where possible. A complete list of primers used is available in Table 2 below. Gene accession numbers are shown according to GenBank.

Western blot: Protein lysates were prepared from activated T-cells in RIPA buffer. Proteins were separated with SDS-PAGE and transferred to a Nylon membrane (GE Healthcare). Membranes were blocked for 2h at room temperature in 5% Milk / TBST, incubated overnight at 4°C with primary antibodies (1:1000) and subsequently with HRP-conjugated secondary antibody (Amersham Bioscience) (1:5000). Antibodies against hexokinase 1, pyruvate kinase M1/2, Aldolase A, Enolase 1 and β-actin were purchased from Cell Signaling; antibodies against Slc16a1 and Slc5a12 were purchased from Abcam.

Lentivirus preparation: Bacterial glycerol stocks containing sh-RNA plasmid clones targeting Slc5a12 were purchased from Sigma and grown in Luria Bertani broth. Plasmids were isolated using Plasmid Maxi kit (Qiagen). HEK293T-cells were grown in 10 x 10cm cell culture dishes to 70% confluence and transfected with plasmids using the calcium phosphate method. The supernatant was harvested 48 and 72 hours after transfection and hundred-fold concentrated in an ultracentrifuge. Aliquots were stored at -80°C.

Lentiviral transduction and sh-RNA-mediated gene silencing: Primary CD4+ T-cells were isolated from C57BL/6 murine lymph nodes and activated with plate bound anti-CD3 and anti-CD28, and IL-2 for 3 days. On day 3, medium was changed and cells were incubated with 25µl virus/10⁶ cells in the presence of polybrene (8µg/ml). Virus was removed 24h later; T-cells were washed twice with PBS and incubated for 24 hours in complete RPMI culture media.

Measurement of lactate, glycolysis, glucose uptake/flux and cell death: Lactate concentration was measured in the synovial fluid or peritoneal lavage using the Lactate assay Kit (Biovision), according to the manufacturer's instructions. Glycolytic metabolism was measured with a Seahorse XF24 Extracellular Flux Analyzer. Briefly, T-cells were grown in high glucose RPMI-1640 supplemented with 10% FCS. One hour before the experiment, 5x10⁵ T-cells were seeded in a 24 well microplate in XF Assay Modified DMEM, and CXCL10, sodium-lactate, metabolic drugs or PBS were injected during measurement. Glucose uptake/flux was measured in T-cells pre-treated with 2-DG, sodium lactate or lactic-acid and then incubated with the fluorescent probes 2-NBDG or 6-NBDG (Life). T-cell viability upon lactate or metabolic drug treatment was assessed by trypan blue exclusion assay.

CTL differentiation and activity assay: Isolated CD8+ T-cells (balb/c) were incubated with CD3-depleted and mytomycin-C-eradicated allogeneic splenocytes (C57BL/6). Differentiated CTLs were enriched with Ficoll and CD3 enrichment kits and co-cultured with endothelial cells (C57BL/6) in the presence or absence of 10mM lactic-acid or sodium-lactate. Dead cells were counted using trypan blue exclusion assay 2, 4, 6 and 18 hours after the start of the assay.

Th subset differentiation: T-cells were isolated from murine lymph nodes and enriched for CD3+ and subsequently CD4+ subsets. 10⁶ cells were plated/well and differentiated towards Th0, Th1, Th2 and Th17 phenotype. Conditions were: Th0 (10ng/ml IL-2); Th1 (10ng/ml IL-2; 3.4ng/ml IL-12; 2µg/ml Anti-IL-4); Th2 (10ng/ml IL-2; 10ng/ml IL-4; 2µg/ml Anti-IFN-y); Th17 (10ng/ml IL-6; 2µg/ml Anti-IL-4; 2µg/ml Anti-IFN-y, 5ng/ml TGF-β). All antibodies and cytokines were purchased from PeproTech.

Intracellular protein staining: Differentiated T-cells were incubated in permeabilization/fixation buffer (ebioscience) overnight at 4°C. Samples were washed in permeabilization buffer (ebioscience) and stained for the cytokines IFN-γ and IL-17, using fluorescently conjugated primary antibodies (1:200, ebiosciences) at 4°C for 30 minutes, and assessed by flow cytometry using a LSR Fortessa (BD Biosciences) and FlowJo version 7.6.5 software.

Human RA synovial tissue collection and immunohistology/immunofluorescence: RA synovial tissue was collected after informed consent (LREC 07/Q0605/29) from a total of 16 RA patients undergoing total joint replacement or ultrasound-guided synovial biopsies as previously described in Humby et al (2009) PLoS Med 6: e1. A summary of the demographical and clinical characteristics of the RA patients is reported in Table 1.

For total T-cell scoring, paraffin sections were stained for CD3 and a semi-quantitative score was applied as previously described in Croia et al (2013) Ann Rheum Dis 72: 1559-1568. For Slc5a12 single and double (with CD4 or CD8) immunofluorescence, after antigen retrieval (S2367, Dako) and block of non-specific binding, slides were incubated with primary antibodies either overnight at 4°C (CD4 and CD8, 1:50, Dako) or 1 hour at RT (Slc5a12, 1:50, Novus Biologicals) followed by fluorochrome-conjugated secondary antibodies (Invitrogen, Eugene, Oregon, USA). All sections were visualised using a Zeiss fluorescence microscope. Quantification was performed by calculating the % of double positive CD4+ Slc5a12⁺ population within the CD4+ or Slc5a12⁺ cells and the % of double positive CD8+ Slc5a12⁺ population within the CD8+ or Slc5a12⁺ cells.

In vivo peritoneal recruitment model: All the in vivo experiments were conducted under the UK Home Office regulation (PPL 70/7443). Activated T-cells (5 x 10⁶/mouse) were pre-treated overnight with Rapamycin (200nM), 2-Deoxyglucose (1mM) or Metformin (2mM), then labelled with the fluorescent cell dye DDAO (Invitrogen) and injected intravenously into syngeneic female C57BL/6 mice that had 3 hours prior received an intraperitoneal injection of CXCL10 (120ng/mouse). 24 hours after injection, mice were sacrificed and spleen and peritoneal lavage were harvested. T-cells were stained for surface markers (CD4 and CD8, ebiosciences) and analysed by FACS. Cells were first gated on CD4 and subsequently analysed for DDAO positivity. This method was used in figure 3G and S3E.

In vivo zymosan-induced peritonitis: C57BL/6 mice were injected in the peritoneal cavity with zymosan (1mg per mouse) on day 0 or left untreated. On day 5, Phloretin (50µM), anti-Slc5a12 antibody (5µg/ml) or anti-rabbit IgG isotype control antibody (5µg/ml; Invitrogen) were injected into the peritoneal cavity. 24 hours later, mice were sacrificed and the peritoneal lavage was harvested. T-cells were stained for surface markers (CD4 and CD8; ebiosciences) and analyzed by FACS. This method was used in figure 6B and S6A-B. Alternatively C57BL/6 mice were injected in the peritoneal cavity with zymosan (1mg per mouse) on day 0. On day 5, activated CD4+ T-cells (5 x 10⁶/mouse) labelled with the fluorescent cell dye CFSE (3.3µM; Invitrogen) were co-injected with anti-Slc5a12 antibody (5µg/ml), phloretin (50µM) or an isotype control antibody (5µg/ml) in the peritoneal cavity. 24 hours later, mice were sacrificed and the peritoneal lavage and the spleen were harvested. T-cells were stained for surface markers (CD4 and CD8; ebiosciences) and analyzed by FACS. Cells were first gated on CD4 and subsequently analysed for CFSE positivity. This method was used in Figures 6C and 13C.

FACS: Isolated T-cells were stained for surface markers; CD3, CD4, CD8, CD25, CXCR3, CCR7, CD62L and LFA-1 with fluorescently conjugated primary antibodies (1:200, ebiosciences) at 4°C for 30 minutes, and assessed by flow cytometry using a LSR Fortessa (BD Biosciences) and FlowJo version 7.6.5 software.

Model of G6PI-induced arthritis: DBA/1 mice purchased from Charles River Laboratories were immunized s.c. with 20µg human G6PI synthetic peptide (hG6PI325-339) (ThermoFisher Scientific) in CFA (Sigma-Aldrich, Taufkirchen, Germany). The indicated amount of peptide was mixed with CFA in a 1:1 ratio (v/v) and emulsified by sonification. For induction of arthritis 100µl of the emulsion was given s.c. at the base of the tail. On day 7 and 11 post induction mice were left untreated or treated sub-plantar into the rear paws with 2µg of antibody; Infliximab (Remicade, Janssen Biologics), anti-TNF (BD bioscience), anti-Slc5a12 (Abcam), Iso-TNF (BD biosciences) and Iso-Slc5a12 (Abcam).

The development of disease was monitored daily by visually assessing the clinical score. A score of 0 indicates no clinical signs of arthritis; a score of 1 for each of the fingers (5 in total), pad and ankle indicates swelling and redness. Maximum score for each paw is 7. A trained observer who was blinded to the immunization status of the mice performed the scoring. n=5 per treatment group. Statistical analysis: Data are expressed as mean ± s.e.m. Two-tailed Student's t-test was used to compare 2 groups with parametric data distribution. For multiple comparison analysis, 1-, 2- or 3-way ANOVA was used. In all case, a p-value of less than 5% was considered to be significant.

**Table 2**

| **Primer name** | **Sequence** | **Species** | **Description** | **Acc-Nr** |
|---|---|---|---|---|
| Slc16a1_F | GCTGGAGGTCCTATCAGCAG | mouse | Monocarboxylic acid transporter 1 | NM_009196.4 |
| Slc16a1_R | AGTTGAAAGCAAGCCCAAGA | mouse | Monocarboxylic acid transporter 1 | |
| Slc5a12_F | AAGCACCTATGAGTACTTACAGC | mouse | sodium-coupled monocarboxylate transporter 2 isoform 1 | NM_001003915.2 |
| Slc5a12_R | ACCAGTCACTTGGTTGAGAGC | mouse | sodium-coupled monocarboxylate transporter 2 isoform 1 | |
| Hk1_F | AAAGCGGTTCAAAGCCAGTG | mouse | hexokinase-1 isoform HK1 | NM_001146100.1 |
| Hk1_R | CACCACAGCTACAATGTTAGCG | mouse | hexokinase-1 isoform HK1 | |
| PkM2_F | CCACTTGCAATTATTTGAGGAA | mouse | Pyruvate Kinase M2 Isoform | NM_011099.3 |
| PkM2_R | GTGAGCAGACCTGCCAGACT | mouse | Pyruvate Kinase M2 Isoform | |
| Glut1_F | CACTGTGGTGTCGCTGTTTG | mouse | Slc2a1 solute carrier family 2 (facilitated glucose transporter), member 1 | NM_011400.3 |
| Glut1_R | ATGGAATAGGACCAGGGCCT | mouse | Slc2a1 solute carrier family 2 (facilitated glucose transporter), member 1 | |
| Glut2_F | GGAAGTCAGGGCAAAGAAAAGC | mouse | Slc2a2 solute carrier family 2, (facilitated glucose transporter) member 2 | NM_031197.2 |
| Glut2_R | AATTGGCATCCGTGAAGAGC | mouse | Slc2a2 solute carrier family 2, (facilitated glucose transporter) member 2 | |
| Glut3_F | AACTTGCTGGCCATCATTGC | mouse | Slc2a3 solute carrier family 2, (facilitated glucose transporter) member 3 | NM_011401.4 |
| Glut3_R | TGCACAGGCCACAGAAAATG | mouse | Slc2a3 solute carrier family 2, (facilitated glucose transporter) member 3 | |
| Glut4_F | TGGCCTTCTTTGAGATTGGC | mouse | Slc2a4 solute carrier family 2 (facilitated glucose transporter), member 4 | NM_009204.2 |
| Glut4_R | AACCCATGCCGACAATGAAG | mouse | Slc2a4 solute carrier family 2 (facilitated glucose transporter), member 4 | |
| Lta_F | TGTGTTCCTGCTCAGTAAGGG | mouse | lymphotoxin-alpha precursor (TNFbeta) | NM_010735.2 |
| Lta_R | ACAGTGCAAAGGCTCCAAAG | mouse | mouse lymphotoxin-alpha precursor (TNFbeta) | |
| IL4_F | TCGGCATTTTGAACGAGGTC | mouse | interleukin-4 precursor | NM_021283.2 |
| IL4_R | TGGTGTTCTTCGTTGCTGTG | mouse | interleukin-4 precursor | |
| IL5_F | CCGCCAAAAAGAGAAGTGTGG | mouse | mouse interleukin-5 precursor | NM_010558.1 |
| IL5_R | TTCCATTGCCCACTCTGTACTC | mouse | interleukin-5 precursor | |
| IFNg_F | ATCAGGCCATCAGCAACAAC | mouse | Interferon gamma precursor | NM_008337.3 |
| IFNg_R | TGCATCCTTTTTCGCCTTGC | mouse | Interferon gamma precursor | |
| IL13_F | ATTGCATGGCCTCTGTAACC | mouse | interleukin-13 precursor | NM_008355.3 |
| IL13_R | GGCGAAACAGTTGCTTTGTG | mouse | interleukin-13 precursor | |
| IL17_F | AAAGCTCAGCGTGTCCAAAC | mouse | interleukin-17A precursor | NM_010552.3 |
| IL17_R | TTCTGGAGCTCACTTTTGCG | mouse | interleukin-17A precursor | |
| Rorc_F | TCAAGTTTGGCCGAATGTCC | mouse | RAR-related orphan receptor gamma | NM_011281.2 |
| Rorc_R | ACTTGTTCCTGTTGCTGCTG | mouse | RAR-related orphan receptor gamma | |
| SLC16A1_F | CACCCACAGAGGCTTTTTGC | human | monocarboxylate transporter 1 | NM_003051.3 |
| SLC16A1_R | GTCGGGCTACCATGTCAACA | human | monocarboxylate transporter 1 | |
| SLC5A12_F | GTGTGCTGTCTTCTCTGGCT | human | sodium-cou pled monocarboxylate transporter 2 | NM_1784983 |
| SLC5A12_R | GCCACAAAAAGTCCTGGCAG | human | sodium-cou pled monocarboxylate transporter 2 | |

## Claims

1. An antibody to solute carrier family 5 member 12 (Slc5a12) for use in the treatment of an inflammatory disease, wherein the antibody is a specific inhibitor of Slc5a12.

2. The antibody for use according to claim 1, wherein the inflammatory disease is selected from the group consisting of rheumatoid arthritis, osteoarthritis, inflammatory bowel disorder, atherosclerosis and psoriasis.

3. An antibody to Slc5a12 in combination with an antibody to solute carrier family 16 member 1 (Slc16a1) for use in the treatment of an inflammatory disease, wherein the antibody to Slc5a12 is a specific inhibitor of Slc5a12 and the antibody to Slc16a1 is a specific inhibitor of Slc16a1.

4. The antibody for use according to claim 3, wherein the inflammatory disease is selected from the group consisting of rheumatoid arthritis, osteoarthritis, inflammatory bowel disorder, atherosclerosis and psoriasis.

5. A kit comprising an antibody to Slc5a12 or an antibody to Slc5a12 in combination with an antibody to Slc16a1, wherein the antibody to Slc5a12 is a specific inhibitor of Slc5a12 and the antibody to Slc16a1 is a specific inhibitor of Slc16a1.

6. The kit according to claim 5 for use in the treatment of an inflammatory disease.

7. The kit for use according to claim 6, wherein the disease is selected from the group consisting of rheumatoid arthritis, osteoarthritis, inflammatory bowel disorder, atherosclerosis and psoriasis.

## Patentansprüche

1. Antikörper gegen Slc5a12 (Solute Carrier Family 5 Member 12) zur Verwendung bei der Behandlung einer Entzündungskrankheit, wobei es sich bei dem Antikörper um einen spezifischen Inhibitor von Slc5a12 handelt.

2. Antikörper zur Verwendung nach Anspruch 1, wobei die Entzündungskrankheit ausgewählt ist aus der Gruppe bestehend aus rheumatoider Arthritis, Osteoarthritis, entzündlicher Darmerkrankung, Atherosklerose und Psoriasis.

3. Antikörper gegen Slc5a12 in Kombination mit einem Antikörper gegen Slc16a1 (Solute Carrier Family 16 Member 1) zur Verwendung bei der Behandlung einer Entzündungskrankheit, wobei es sich bei dem Antikörper gegen Slc5a12 um einen spezifischen Inhibitor von Slc5a12 und bei dem Antikörper gegen Slc16a1 um einen spezifischen Inhibitor von Slc16a1 handelt.

4. Antikörper zur Verwendung nach Anspruch 3, wobei die Entzündungskrankheit ausgewählt ist aus der Gruppe bestehend aus rheumatoider Arthritis, Osteoarthritis, entzündlicher Darmerkrankung, Atherosklerose und Psoriasis.

5. Kit, umfassend einen Antikörper gegen Slc5a12 oder einen Antikörper gegen Slc5a12 in Kombination mit einem Antikörper gegen Slc16a1, wobei es sich bei dem Antikörper gegen Slc5a12 um einen spezifischen Inhibitor von Slc5a12 und bei dem Antikörper gegen Slc16a1 um einen spezifischen Inhibitor von Slc16a1 handelt.

6. Kit nach Anspruch 5 zur Verwendung bei der Behandlung einer Entzündungskrankheit.

7. Kit zur Verwendung nach Anspruch 6, wobei die Krankheit ausgewählt ist aus der Gruppe bestehend aus rheumatoider Arthritis, Osteoarthritis, entzündlicher Darmerkrankung, Atherosklerose und Psoriasis.

## Revendications

1. Anticorps contre le membre 12 de la famille de porteur de soluté 5 (Slc5a12) pour utilisation dans le traitement d'une maladie inflammatoire, où l'anticorps est un inhibiteur spécifique de Slc5a12.

2. Anticorps pour utilisation selon la revendication 1, où la maladie inflammatoire est choisie dans le groupe constitué de la polyarthrite rhumatoïde, l'arthrose, un trouble intestinal inflammatoire, l'athérosclérose et le psoriasis.

3. Anticorps contre Slc5a12 en combinaison avec un anticorps contre le membre 1 de la famille de porteur de soluté 16 (Slc16a1) pour utilisation dans le traitement d'une maladie inflammatoire, où l'anticorps contre Slc5a12 est un inhibiteur spécifique de Slc5a12 et l'anticorps contre Slc16a1 est un inhibiteur spécifique de Slc16a1.

4. Anticorps pour utilisation selon la revendication 3, où la maladie inflammatoire est choisie dans le groupe constitué de la polyarthrite rhumatoïde, l'arthrose, un trouble intestinal inflammatoire, l'athérosclérose et le psoriasis.

5. Kit comprenant un anticorps contre Slc5a12 ou un anticorps contre Slc5a12 en combinaison avec un anticorps contre Slc16a1, dans lequel l'anticorps contre Slc5a12 est un inhibiteur spécifique de Slc5a12 et l'anticorps contre Slc16a1 est un inhibiteur spécifique de Slc16a1.

6. Kit selon la revendication 5 pour utilisation dans le traitement d'une maladie inflammatoire.

7. Kit pour utilisation selon la revendication 6, dans lequel la maladie est choisie dans le groupe constitué de la polyarthrite rhumatoïde, l'arthrose, un trouble intestinal inflammatoire, l'athérosclérose et le psoriasis.
